# EUROPEAN PATENT SPECIFICATION

(11) **EP 2 923 761 B1**
(45) Date of publication and mention of the grant of the patent: **04.01.2017**
(21) Application number: 15160511.0
(22) Date of filing: 24.03.2015
(51) Int. Cl.: B01L 3/00, B01L 7/00, G01N 35/00

(54) **NUCLEIC ACID AMPLIFICATION REACTION DEVICE AND NUCLEIC ACID AMPLIFICATION METHOD**
VORRICHTUNG ZUR NUKLEINSÄUREAMPLIFIKATIONSREAKTION UND NUKLEINSÄUREAMPLIKATIONSVERFAHREN
DISPOSITIF ET PROCÉDÉ D'AMPLIFICATION D'ACIDE NUCLÉIQUE

(30) Priority: 26.03.2014 JP 2014063242
(43) Date of publication of application: 30.09.2015
(73) Proprietor: Seiko Epson Corporation, Shinjuku-ku Tokyo 163-0811 (JP)
(72) Inventor: Koeda, Hiroshi, Nagano, 392-8502 (JP)
(74) Representative: Hoffmann Eitle

(56) References cited:
- EP-A1- 2 145 949
- EP-A1- 2 778 687
- WO-A1-2010/147561
- US-A1- 2011 256 590

## Description

### BACKGROUND

### 1. Technical Field

The present invention relates to a nucleic acid amplification reaction device and a nucleic acid amplification method.

### 2. Related Art

In recent years, with the advances in techniques using genes, medical treatment using genes, such as gene diagnosis or gene therapy, has been attracting attention. In the field of agriculture and livestock, many techniques using genes have been developed for breed identification and breeding. As a technique for using genes, a technique, such as PCR (Polymerase Chain Reaction), has widely been used. Nowadays, PCR is an essential technique for revealing information of biological substances. In PCR, a thermal cycle is performed on a solution containing a nucleic acid to be amplified (target nucleic acid) and a reagent to amplify the target nucleic acid. Generally, the thermal cycle for PCR is performed at two or three different temperatures.

A kit for a simple test, such as immunochromatography, has been the mainstream for the current diagnosis of an infectious disease including influenza in medical practices. Such a simple test, however, often suffers from poor accuracy and it has thus been desired to apply PCR, which can be expected to provide diagnosis with higher accuracy, to the diagnosis of infectious diseases. Furthermore, only a short period of time can be used for the medical check in general outpatient practices in a medical facility because of the limited time available for clinical examination. For this reason, current examination of, for example, influenza is performed rapidly using a simple examination, such as immunochromatography, at the expense of the accuracy of examination.

Under such circumstances, in the field of medical practices, it is necessary to reduce the time required for reaction in order to realize PCR examination which can be expected to provide a result with higher accuracy. As a device for achieving a PCR reaction in a short period of time, for example, JP-A-2009-136250 discloses a biological sample reaction device which performs a thermal cycle by rotating a biological sample reaction chip filled with a reaction solution and a liquid, which is not mixed with the reaction solution and has specific gravity smaller than the reaction solution, around a rotation axis in a horizontal direction to move the reaction solution (JP-A-2009-136250). As a technique of PCR, some methods are disclosed, such as a method using magnetic particles (JP-A-2009-207459) and a method of performing a thermal cycle for PCR using magnetic particles as a tool for moving droplets, which are moved in a region on a substrate where the temperature is changed (JP-A-2008-012490).

In a nucleic acid extraction method using magnetic particles, when detaching nucleic acid from the surface of the magnetic particles to a solution (elution solution), if the elution solution is heated, elution efficiency is enhanced. EP 2 778 687 A1 discloses a cartridge for nucleic acid amplification reaction including a tube that has a first plug formed on a first oil, a second plug formed of a first washing solution which undergoes phase operation when being mixed with oil and washes nucleic acid-binding solid-phase carriers having bound to a nucleic acid, a third plug formed of a second oil, a fourth plug formed of an eluate which undergoes phase separation when being mixed with oil and causes the nucleic acid to be eluted from the nucleic acid-binding solid-phase carriers having bound to the nucleic acid, a fifth plug formed of a third oil, a sixth plug formed of a nucleic acid amplification reaction solution which undergoes phase separation when being mixed with oil and causes a nucleic acid amplification reaction, and a seventh plug formed of a fourth oil in this order in the inside of the tube.

Further, US 2011/0256590 A1 teaches a biochip including a chamber that has a longitudinal direction, a holding unit that holds a liquid sample within a predetermined area of the chamber provided along the longitudinal direction, and releases the liquid sample from the predetermined area to an area inside the chamber by using a predetermined pressing force; and a pressed member that applies the predetermined pressing force to the liquid sample.

Furthermore, WO 2010/147561 A1 teaches a method for conducting at least two reactions in a reaction tube, the method comprising the steps of providing at least two reaction phases within the reaction tube for allowing the reactions to occur therein, providing a separation phase that is immiscible with the two reaction phases and which is disposed therebetween, providing at least one particle capable of being coupled to a chemical species, wherein the particle is moveable between the reaction phases to introduce the chemical spaces thereto.

Moreover, EP 2 145 949 A1 teaches a temperature cooling apparatus and a temperature cooling method which can arrest foaming and inflation of the air contained in a liquid and can perform temperature controlling of a liquid of high reliance, and is constructed to have one or a plurality of sets of nozzles which can suck and discharge a liquid through a tip, and can retain the sucked liquid, a suction and discharge mechanism which can suck and discharge a gas via each nozzle, a temperature regulator which can maintain one or two or more set predetermined temperatures for a predetermined time regarding one or two or more predetermined temperature controlling regions set in the each nozzle, a movement mechanism which allows relative movement between one or two or more containers which can accommodate a liquid and a nozzle, and a controlling part which instructs a movement mechanism, the suction and discharge mechanism or the temperature regulator to adjust a liquid to be sucked into the nozzle and a liquid amount thereof, a position of the sucked liquid in the nozzle and temperature controlling of the liquid.

### SUMMARY

An advantage of some aspects of the invention is to provide a nucleic acid amplification reaction device capable of heating an elution solution at a stable temperature. The invention relates to the nucleic acid amplification reaction device of claim 1 and the method of amplifying nucleic acid of claim 6. The remaining claims are directed to preferred embodiments.

A nucleic acid amplification reaction device according to an aspect includes a rotary body on which a cartridge is mountable, the cartridge including a tube having, in this order in the inside thereof, a first plug formed of oil, a second plug formed of a first washing solution which undergoes phase separation from oil and washes magnetic particles bound to nucleic acid, a third plug formed of oil, a fourth plug formed of an elution solution which undergoes phase separation from oil and causes the nucleic acid to be eluted from the magnetic particles bound to the nucleic acid, and a fifth plug formed of oil, and a nucleic acid amplification reaction container which communicates with the side of the tube on which the fifth plug is disposed and in which a nucleic acid amplification reaction reagent is held in oil, and a first heater which is able to heat the end portion of the tube on the side communicating with the nucleic acid amplification reaction container and a first region as the end portion of the nucleic acid amplification reaction container on the side communicating with the tube. The posture of the cartridge is changed by the rotation of the rotary body, and droplets containing the elution solution introduced from the tube move in the inside of the nucleic acid amplification reaction container. The nucleic acid amplification reaction device may further include a second heater which is able to heat a second region as the other end portion of the nucleic acid amplification reaction container. The nucleic acid amplification reaction device may further include a control unit which controls the temperature of the first heater such that the temperature of the first heater is set to a first temperature during the elution of the nucleic acid and the temperature of the first heater becomes a second temperature higher than the first temperature after the elution of the nucleic acid before the posture of the cartridge is changed by the rotation of the rotary body and droplets containing the elution solution introduced from the tube into the nucleic acid amplification reaction container move to the second region.

A method of amplifying a nucleic acid amplification reaction according to another aspect is a method of amplifying a nucleic acid amplification reaction using the nucleic acid amplification reaction device, and the method includes causing the nucleic acid to be eluted from magnetic particles bound to the nucleic acid in a fourth plug formed of an elution solution, retracting the magnetic particles from the fourth plug toward the first plug after the elution of the nucleic acid, and performing a droplet forming process to push out the elution solution from the tube to the nucleic acid amplification reaction container after the retraction of the magnetic particles. The temperature of the first heater is set to a first temperature during the elution of the nucleic acid, and the temperature of the first heater is changed to a second temperature such that the temperature of the first heater becomes the second temperature after the elution of the nucleic acid before the posture of the cartridge is changed by the rotation of the rotary body and droplets containing the elution solution inside the nucleic acid amplification reaction container move from a first region to a second region. While the retraction of the magnetic particles or the droplet forming process is performed, the temperature of the first heater may be increased from the first temperature to the second temperature. The nucleic acid may be RNA, and the elution solution may contain a reverse transcription reaction reagent, and the method further includes performing reverse transcription to cause the RNA to be reversely transcribed in the elution solution. While the retraction of the magnetic particles, the droplet forming process, or the reverse transcription is performed, the temperature of the first heater may be increased from the first temperature to the second temperature.

### BRIEF DESCRIPTION OF THE DRAWINGS

The invention will be described with reference to the accompanying drawings, wherein like numbers reference like elements.
Figs. 1A and 1B are diagrams illustrating a cartridge.
Figs. 2A to 2C are diagrams illustrating the operation of the cartridge.
Figs. 3A to 3D are diagrams illustrating a tank.
Fig. 4 is a diagram illustrating a fixing claw, a guide plate, and a mounting portion.
Figs. 5A and 5B are diagrams illustrating the periphery of a PCR container.
Fig. 6A is a perspective view of the internal configuration of a PCR device. Fig. 6B is a side view of the main configuration of the PCR device.
Fig. 7 is a block diagram of the PCR device.
Fig. 8A is a diagram illustrating a rotary body. Fig. 8B is a diagram illustrating a state where the cartridge is mounted in the mounting portion of the rotary body.
Figs. 9A to 9D are diagrams illustrating a state of the PCR device when the cartridge is mounted.
Fig. 10 is a conceptual diagram of the behavior of magnetic particles when magnets are moved downward.
Figs. 11A to 11C are diagrams illustrating a nucleic acid elution process.
Fig. 12 is a conceptual diagram of the behavior of the magnetic particles when the magnets oscillate.
Fig. 13 is a table showing the presence/absence of oscillation of the magnets.
Figs. 14A to 14C are diagrams illustrating a droplet forming process.
Figs. 15A to 15D are diagrams illustrating a thermal cycle process.
Figs. 16A and 16B are diagrams illustrating a cartridge of a second embodiment.
Fig. 17A is a diagram illustrating the initial state of a cartridge of the second embodiment. Fig. 17B is a side view showing a state where a plunger is pushed in the state of Fig. 17A and a seal comes into contact with a lower syringe. Fig. 17C is a diagram illustrating the cartridge of the second embodiment after the plunger is pushed.
Figs. 18A and 18B are diagrams showing the periphery of a PCR container of the second embodiment.

### DESCRIPTION OF EXEMPLARY EMBODIMENTS

Hereinafter, embodiments of the invention will be described in detail. The description of the specification makes those skilled in the art clearly see the objects, features, advantages, and ideas of the invention, and those skilled in the art can easily make the invention based on the description of the specification. The embodiments of the invention described below are preferred embodiments of the invention and are only for illustration and description, and the invention is not limited thereto. Those skilled in the art know for sure that the invention can be changed and modified in various ways based on the description of the specification within the intention and the scope of the invention described in the specification.

### First Embodiment

First, a cartridge which is mounted in a PCR device 50 (nucleic acid amplification reaction device) will be described, and then the configuration and operation of the PCR device 50 of this embodiment will be described.

### Cartridge 1

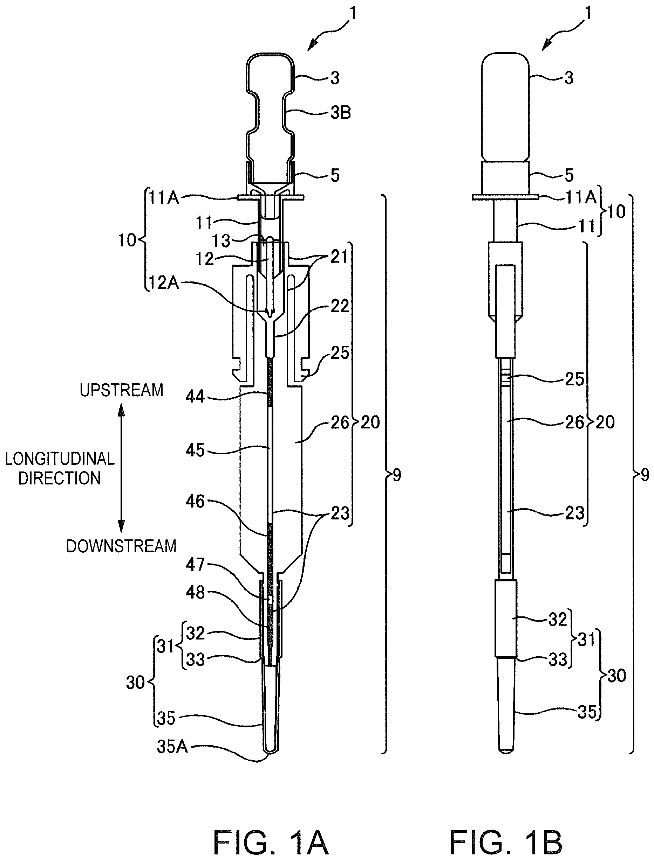

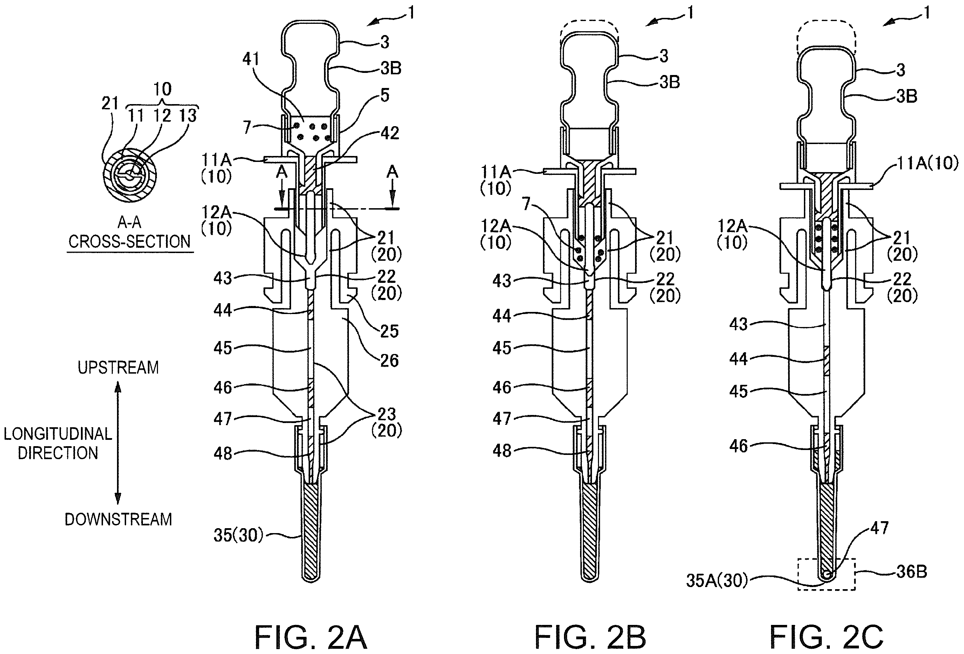

Figs. 1A and 1B are diagrams illustrating a cartridge 1. Figs. 2A to 2C are diagrams illustrating the operation of the cartridge 1. Fig. 2A is a diagram illustrating the initial state of the cartridge 1. Fig. 2B is a side view showing a state where a plunger 10 is pushed in the state of Fig. 2A such that a seal 12A comes into contact with a lower syringe 22. Fig. 2C is a diagram illustrating the cartridge 1 after the plunger 10 is pushed.

The cartridge 1 is a container in which a nucleic acid elution process for causing nucleic acid to be eluted from magnetic particles 7 bound to the nucleic acid is performed. The cartridge 1 is also a container in which a thermal cycle process for a polymerase reaction is performed on an elution solution 47 to be a PCR solution.

A nucleic acid extraction process is performed in a tank 3, and the nucleic acid is purified while passing through a tube 20. The material of the tube 20 is not particularly limited, and the tube can be made of, for example, glass, resin, such as plastic, metal, or the like. In particular, it is preferable to select transparent glass or resin as the material of the tube 20 since the inside of the tube 20 can be observed from the outside. It is preferable to select a material which transmits a magnetic force or a nonmagnetic material as the material of the tube 20 since this makes it easy to move magnetic particles by applying a magnetic force from the outside of the tube 20 so as to pass the magnetic particles through the tube 20. It is preferable that the material of the tube has heat resistance to a temperature of at least 100°C since a heater (a first heater 65A described below) is disposed near the tube. The tube 20 and the tank may be made of the same material.

The tube 20 has a washing solution plug 45, an elution solution plug 47, and an oil plug. Since the magnetic particles 7 bound to the nucleic acid are attracted to a magnet outside, if the magnet is moved outside along the tube 20, the magnetic particles 7 move inside the tube 20, pass through the washing solution plug 45, and reach the elution solution plug 47. The nucleic acid bound to the magnetic particles 7 is washed with the washing solution in the washing solution plug 45 and eluted in the elution solution plug 47. The "plug" means a liquid when a certain liquid occupies a section in the tube 20. For example, in Figs. 2A to 2C, a liquid held in the form of a column inside a capillary 23 is called a "plug". Oil undergoes phase separation from other liquids (oil is not mixed with other liquids). Therefore, a plug formed of oil functions to prevent water-soluble plugs on both sides of the tube from being mixed with each other. Although it is preferable that air bubbles or other liquids are not present in a plug or between plugs, air bubbles or other liquids may be present as long as the magnetic particles 7 can pass through the plug.

The type of oil is not particularly limited, and mineral oil, silicone oil (such as 2CS silicone oil or the like), plant oil, or the like can be used. However, if oil having higher viscosity is used, when the nucleic acid-binding solid-phase carriers are moved in the interface between the oil and the upper plug, a "wiping effect" produced by the oil can be enhanced. With this, when the nucleic acid-binding solid-phase carriers are moved from the upper plug to the plug formed of the oil, it is possible to make it more difficult for water-soluble components, which are adhered to the nucleic acid-binding solid-phase carriers, to be brought into the oil.

The thermal cycle process is performed in a PCR container 30 of the cartridge 1. The PCR container 30 is filled with oil, and the elution solution 47 undergoes phase separation from the oil. Therefore, when the elution solution plug 47 is pushed out from the tube 20 to the PCR container 30, the elution solution plug 47 becomes a droplet. The elution solution 47 in the form of a droplet precipitates since the specific gravity thereof is greater than that of the oil. If a high temperature region 36A and a low temperature region 36B are formed in the PCR container 30 by an external heater, and the entire cartridge 1 is repeatedly moved upside down together with the heater, the elution solution 47 in the form of a droplet alternately moves between the high temperature region 36A and the low temperature region 36B, whereby a two-stage temperature process is performed on the elution solution 47 as a PCR solution.

The material of the PCR container 30 is not particularly limited, and the PCR container 30 is formed of, for example, glass, resin, such as plastic, metal, or the like. It is preferable that the material of the PCR container 30 has heat resistance to a temperature of at least 100°C since the first heater 65A is disposed near the container. It is preferable to select a transparent or semitransparent material as the material of the PCR container 30 since this makes it easy to perform fluorescence measurement (luminance measurement). However, the entire region of the PCR container 30 does not need to be transparent or semitransparent, and at least the site (for example, a bottom 35A of the PCR container 30) facing a fluorescence measuring instrument 55 may be transparent or semitransparent. The PCR container 30 and the tank 3 or the plunger 10 may be made of the same material.

The cartridge 1 is constituted by the tank 3 and a cartridge main body 9. In a kit constituting the cartridge 1, an adapter 5 is prepared in advance together with the tank 3 and the cartridge main body 9. The tank 3 and the cartridge main body 9 are connected to each other through the adapter 5, whereby the cartridge 1 is assembled. However, the tank 3 may be directly attached to the cartridge main body 9.

In the following description of the constituents of the cartridge 1, as shown in Fig. 2A, the direction along the long cartridge 1 is referred to as a "longitudinal direction", the tank 3 side is referred to as an "upstream side", and the PCR container 30 side is referred to as a "downstream side". The upstream side is simply referred to as an "upper side", and the downstream side is simply referred to as a "lower side".

### 1. Tank

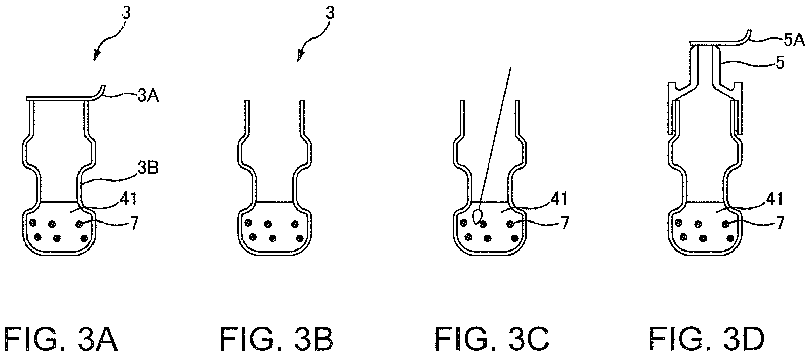

Figs. 3A to 3D are diagrams illustrating the tank 3.

The tank 3 prepared in advance in the kit contains a dissolution solution 41 and magnetic particles 7. A detachable lid 3A is attached to the opening of the tank 3 (see Fig. 3A). As the dissolution solution 41, 5 M guanidine thiocyanate, 2% Triton X-100, and 50 mM Tris-HCl (pH 7.2) are used. An operator removes the lid 3A to open the opening of the tank 3 (see Fig. 3B), and dips a cotton swab smeared with a virus into the dissolution solution 41 in the tank 3 to collect the virus in the dissolution solution 41 (see Fig. 3C). When the liquid in the tank 3 is stirred, the tank 3 may be shaken in the state of Fig. 3C. In this case, however, the dissolution solution 41 easily overflows. Accordingly, as shown in Fig. 3D, it is preferable to shake the tank 3 after the adapter 5 covered with a lid 5A is attached to the opening of the tank 3. In this way, the substance in the tank 3 is stirred, and the virus particles are dissolved by the dissolution solution 41, whereby the nucleic acid is liberated, and silica coated on the magnetic particles 7 adsorbs the nucleic acid. The magnetic particles 7 correspond to the nucleic acid-binding solid-phase carriers. Thereafter, the operator detaches the lid 5A of the adapter 5 attached to the opening of the tank 3 and attaches the tank 3 to the cartridge main body 9 through the adapter 5 (see Fig. 2A).

The tank 3 is made of flexible resin and is dilatable. When the state of the plunger 10 slides and the state of the cartridge changes from the state of Fig. 2A to the state of Fig. 2B, the tank 3 dilates, and this prevents the pressure in the tube 20 from excessively increasing and prevents the liquid in the tube 20 from being pushed out to the downstream side. It is possible to form a deformation portion 3B in the tank 3 such that the tank 3 easily dilates.

A sample used for extracting and amplifying nucleic acid is not limited to viruses and may be cells. The sources of cells are not particularly limited and may be microorganisms, tissue fragments or blood of higher organisms, and the like.

The dissolution solution 41 is not particularly limited as long as the solution contains a chaotropic substance, and may contain a surfactant to destroy the cell membrane or denature proteins contained in the cells. The surfactant is not particularly limited as long as the surfactant is generally used for extracting nucleic acid from cells and the like. Specific examples thereof include nonionic surfactants including triton-based surfactants, such as Triton-X or tween-based surfactants such as Tween-20, and anionic surfactants such as N-lauroylsarcosine sodium (SDS). It is particularly preferable to use nonionic surfactants at a concentration within a range of 0.1% to 2%. It is preferable for the dissolution solution to contain a reductant such as 2-mercaptoethanol or dithiothreitol. The dissolution solution may be a buffer solution, and pH thereof is preferably neutral such as pH 6 to 8. Considering the above, specifically, it is preferable for the dissolution solution to contain 3 M to 7 M of a guanidine salt, 0% to 5% of a nonionic surfactant, 0 mM to 0.2 mM of EDTA, 0 M to 0.2 M of a reductant, and the like.

The chaotropic substance acts to generate chaotropic ions (monovalent anions having a large ion radius) in an aqueous solution and enhance water solubility of hydrophobic molecules. This substance is not particularly limited as long as the substance contributes to the adsorption of nucleic acid onto the solid-phase carriers. Specific examples thereof include guanidine thiocynate, guanidine hydrochloride, sodium iodide, potassium iodide, sodium perchlorate, and the like. Among these, guanidine thiocynate or guanidine hydrochloride having a potent protein denaturating action is preferable. The concentration of these chaotropic substances used varies with the type of each of the substances. For example, when guanidine thiocyanate is used, this substance is preferably used in a range of 3 M to 5.5 M, and when guanidine hydrochloride is used, this substance is preferably used at a concentration of 5 M or more.

The tool for collecting the sample is not particularly limited, and instead of a cotton swab, a spatula, a rod, a scraper, or the like may be selected according to the purpose.

The internal volume of the tank 3 is not particularly limited, and may be, for example, from 0.1 mL to 100 mL. The material of the tank 3 is not particularly limited, and the tank 3 can be made of, for example, glass, resin, such as plastic, metal, or the like. Particularly, it is preferable to select transparent glass or resin as the material of the tank since the inside of the tank 3 can be observed from the outside. The tank 3 and each tube 20 may be formed by monolithic molding or may be detachable from each other. If a flexible material, such as rubber, elastomer, and polymer are used as the material of the tank 3, it is possible to increase the internal pressure of the tank 3 by deforming the tank 3 in a state where the tank 3 is covered with a lid. As a result, it is possible to push the content of the tube 20 out from the tip of the tube toward the outside from the inside of the tube.

### 2. Cartridge Main Body

The cartridge main body 9 has the plunger 10, the tube 20, and the PCR container 30.

### 2-1. Plunger

Hereinafter, the plunger 10 will be described referring to Figs. 2A to 2C.

The plunger 10 is a movable plunger which pushes out a liquid from the downstream side of the tube 20 functioning as a syringe. The plunger 10 functions to push out a predetermined amount of liquid in the tube 20 from the terminal of the tube 20 to the PCR container 30. The plunger 10 also functions to attach the tank 3 through the adapter 5.

The plunger 10 has a cylindrical portion 11 and a rod-like portion 12. The cylindrical portion 11 is provided on the tank 3 side (upstream side), and the rod-like portion 12 is provided on the tube 20 side (downstream side). The rod-like portion 12 is supported by two plate-like ribs 13 from the inner wall on the downstream side of the cylindrical portion 11. The downstream side of the rod-like portion 12 protrudes from the cylindrical portion 11 toward the downstream side.

The cylindrical portion 11 is opened toward the upstream side and the downstream side, and the inner wall of the cylindrical portion 11 becomes a path of a liquid. The adapter 5 fits to the opening on the upstream side (tank 3 side) of the cylindrical portion 11. In the plunger 10 of the cartridge main body 9 prepared in advance in the kit, a detachable lid may be attached to the opening on the upstream side of the cylindrical portion 11. The opening on the downstream side of the cylindrical portion 11 is positioned in the inside of an upper syringe 21 of the tube 20. The magnetic particles 7 introduced from the opening on the upstream side of the cylindrical portion 11 pass through the inside of the cylindrical portion 11, escape from the inside and outside of the ribs 13, come out from the opening on the downstream side of the cylindrical portion 11, and are introduced into the upper syringe 21 of the tube 20.

The downstream side of the cylindrical portion 11 fits to the inner wall of the upper syringe 21 of the tube 20. The cylindrical portion 11 can slide relative to the upper syringe 21 in the longitudinal direction while coming into contact with the inside of the upper syringe 21 of the tube 20.

In the periphery of the opening on the upstream side of the cylindrical portion 11, a mounting board 11A for mounting the adapter 5 is formed. The mounting board 11A also has a portion which is pushed when the plunger 10 is pushed. If the mounting board 11A is pushed, the plunger 10 slides relative to the tube 20, whereby the state of the cartridge changes from the state of Fig. 2A to the state of Fig. 2C. If the plunger 10 moves to the downstream side, the mounting board 11A comes into contact with the upper rim of the tube 20 (see Fig. 2C). That is, the interval between the mounting board 11A of the plunger 10 and the upper rim of the tube 20 becomes the sliding length of the plunger 10.

In the initial state, the rod-like portion 12 is positioned in the inside of the upper syringe 21 of the tube 20 and is separated from a lower syringe 22 (see Fig. 2A). If the plunger 10 slides relative to the tube 20, the rod-like portion 12 is inserted into the lower syringe 22 of the tube 20 and slides relative to the lower syringe 22 in the downstream direction while coming into contact with the inside of the lower syringe 22 (see Figs. 2B and 2C).

The cross-section of the rod-like portion 12 perpendicular to the longitudinal direction has a circular shape. However, the cross-sectional shape of the rod-like portion 12 may be circular, oval, or polygonal and is not particularly limited as long as the rod-like portion 12 can fit to the inner wall of the lower syringe 22 of the tube 20.

The seal 12A is formed in the end portion on the downstream side of the rod-like portion 12. If the seal 12A fits into the lower syringe 22, the liquid in the tube 20 on the downstream side is prevented from flowing back toward the upper syringe 21. Then, if the plunger 10 is pushed from the state of Fig. 2B to the state of Fig. 2C, the liquid in the tube 20 is pushed out from the downstream side by the amount corresponding to the volume in which the seal 12A slides inside the lower syringe 22.

The volume in which the seal 12A slides inside the lower syringe 22 (the amount of the liquid in the tube 20 pushed out from the downstream side) is greater than the total volume of the elution solution plug 47 and a third oil plug 48 in the tube 20. With this, the liquid in the tube 20 can be pushed out such that the elution solution 47 does not remain in the tube 20.

The material of the plunger 10 is not particularly limited and can be made of, for example, glass, resin, such as plastic, metal, or the like. The cylindrical portion 11 and the rod-like portion 12 of the plunger 10 may be formed of the same material integrally, or may be formed of different materials. Here, the cylindrical portion 11 and the rod-like portion 12 are separately molded with resin, and the cylindrical portion 11 and the rod-like portion 12 are bonded to each other through the ribs 13, whereby the plunger 10 is formed.

The plunger 10 accommodates oil 42 and a first washing solution 43 in advance in the inside thereof. The specific gravity of the oil 42 in the plunger 10 is smaller than that of the first washing solution 43. Therefore, when the tank 3 is attached to the cartridge main body 9, if the cartridge main body 9 is made to stand such that the mounting board 11A of the plunger 10 becomes the upper side, as shown in Fig. 2A, the oil 42 is disposed between the liquid in the tank 3 and the first washing solution 43 of the cartridge main body 9. As the oil 42, 2CS silicone oil is used, and as the first washing solution 43, 8M guanidine hydrochloride and 0.7% Triton X-100 are used.

The first washing solution 43 may be a liquid which undergoes phase separation when being mixed with any of the oil 42 and oil 44. It is preferable that the first washing solution 43 is water or a low salt-concentration aqueous solution, and the low salt-concentration aqueous solution is preferably a buffer solution. The salt concentration of the low salt-concentration aqueous solution is preferably equal to or less than 100 mM, more preferably equal to or less than 50 mM, and most preferably equal to or less than 10 mM. The lower limit of the low salt-concentration aqueous solution is not particularly limited, but is preferably equal to or greater than 0.1 mM, more preferably equal to or greater than 0.5 mM, and most preferably equal to or greater than 1 mM. This solution may also contain a surfactant, such as Triton, Tween, or SDS, and pH thereof is not particularly limited. The type of salt for making the buffer solution is not particularly limited, and salts, such as TRIS, HEPES, PIPES, and phosphoric acid, are preferably used. It is preferable that the washing solution contains alcohol in such an amount that does not hinder the adsorption of nucleic acid onto carriers, a reverse transcription reaction, a PCR reaction, and the like. In this case, the concentration of alcohol is not particularly limited, and may be equal to or less than 70%, equal to or less than 60%, equal to or less than 50%, equal to or less than 40%, equal to or less than 30%, equal to or less than 20%, and equal to or less than 10%. However, the concentration of alcohol is preferably equal to or less than 5% or equal to or less than 2%, more preferably equal to or less than 1% or equal to or less than 0.5%, and most preferably equal to or less than 0.2% or equal to or less than 0.1%.

The first washing solution 43 may contain a chaotropic agent. For example, if the first washing solution 43 contains guanidine hydrochloride, it is possible to wash particles and the like while maintaining or reinforcing the adsorption state of nucleic acid adsorbed onto the particles and the like. When the first washing solution contains guanidine hydrochloride, the concentration of this agent can be controlled, for example, to be equal to or greater than 3 mol/L and equal to or less than 10 mol/L, and preferably equal to or greater than 5 mol/L and equal to or less than 8 mol/L. If the concentration of guanidine hydrochloride is within the above range, it is possible to wash off foreign substances and the like while further stabilizing the adsorption of nucleic acid adsorbed onto particles and the like.

### 2-2. Tube

Hereinafter, the tube 20 will be described with reference to Figs. 2A to 2C.

The tube 20 has the shape of a cylinder through which a liquid can flow in the longitudinal direction. The tube 20 has the upper syringe 21, the lower syringe 22, and a capillary 23, and the inner diameter of each of these portions is different in a stepwise manner.

The upper syringe 21 has the shape of a cylinder through which a liquid can flow in the longitudinal direction. The cylindrical portion 11 of the plunger 10 slidably comes into contact with the inside of the inner wall of the upper syringe 21. The upper syringe 21 functions as a syringe for the cylindrical portion 11 of the plunger 10.

The lower syringe 22 has the shape of a cylinder through which a liquid can flow in the longitudinal direction. The seal 12A of the rod-like portion 12 of the plunger 10 can slidably fit to the inner wall of the lower syringe 22. The lower syringe 22 functions as a syringe for the rod-like portion 12 of the plunger 10.

The capillary 23 has the shape of a capillary tube through which a liquid can flow in the longitudinal direction. The inner diameter of the capillary 23 has such a size that enables the liquid to be maintained in the form of a plug. Here, the inner diameter is 1.0 mm. The inner diameter of the terminal (the terminal on the downstream side of the tube 20) of the capillary 23 is 0.5 mm which is smaller than the above-described diameter. The inner diameter of the terminal of the capillary 23 is set to be smaller than the diameter (1.5 mm to 2.0 mm) of the elution solution, which will be described below, in the form of a droplet. Accordingly, when the elution solution plug 47 is pushed out from the terminal of the capillary 23, the elution solution in the form of a droplet can be inhibited from being adhered to the terminal of the capillary 23 or flowing back into the capillary 23.

The capillary 23 may have a cavity in the inside thereof and have the shape of a cylinder through which a liquid can flow in the longitudinal direction. The capillary 23 may be curved in the longitudinal direction, but it is preferable that the capillary 23 is straight. The internal cavity of the tube is not particularly limited in terms of size and shape as long as a liquid can be maintained in the form of a plug inside the tube. The size of the internal cavity of the tube or the shape of a cross-section thereof perpendicular to the longitudinal direction may be changed along the longitudinal direction of the tube.

The shape of the cross-section of the tube that is perpendicular to the longitudinal direction of the exterior of the tube is not limited. The thickness (a distance between the sidewall of the internal cavity and the exterior surface) of the tube is not particularly limited. When the tube has the shape of a cylinder, the inner diameter (the diameter of a circle which is a cross-section perpendicular to the longitudinal direction of the internal cavity) can be set to, for example, 0.5 mm to 2 mm. If the inner diameter of the tube is within the above range, it is easy to form a plug of a liquid within a wide range of tube materials and liquid types. It is preferable that the tube is tapered toward the tip, and the diameter of the tip can be set to 0.2 mm to 1 mm. If the inner diameter of the terminal of the capillary 23 (the diameter of the opening of the capillary 23) is set to be small, it is possible to inhibit the elution solution 47, which has been formed in a droplet in the PCR container 30, from being adsorbed onto the opening of the capillary 23 and becoming inseparable. However, if the inner diameter of terminal of the capillary 23 is too small, a large number of small droplets of the elution solution 47 are formed. In the capillary 23, if the diameter of a portion other than the terminal is made small just like the terminal, it is not desirable since the cartridge 1 will be lengthened to secure the volume of each plug.

In the inside of the capillary 23, a first oil plug 44, the washing solution plug 45, a second oil plug 46, the elution solution plug 47, and the third oil plug 48 are provided in order from the upstream side. That is, oil plugs are disposed on both sides of a water-soluble plug (the washing solution plug 45 or the elution solution plug 47).

In the upper syringe 21 and the lower syringe 22 on the upstream side from the first oil plug 44, the oil 42 and the washing solution 43 are accommodated in advance (see Fig. 2A). The inner diameter of the upper syringe 21 and the lower syringe 22 is greater than the inner diameter of the capillary 23. In the upper syringe 21 and the lower syringe 22, the liquid (the oil 42 and the washing solution 43) cannot be maintained in the form of a column just like a plug. However, since the first oil plug 44 is held in the form of a plug by the capillary 23, the oil constituting the first oil plug 44 is inhibited from moving to the upstream side.

The washing solution plug 45 may be formed of 5 mM of a tris-hydrochloric acid buffer solution which is a second washing solution. However, the second washing solution may basically have the same configuration as described in the first washing solution. The second washing solution may be the same as or different from the first washing solution. A solution which does not substantially contain a chaotropic substance is preferably used. This is to prevent a chaotropic substance from being brought into the later solution. As described above, it is preferable that this washing solution contains alcohol in such an amount that does not hinder the adsorption of nucleic acid onto carriers, a reverse transcription reaction, a PCR reaction, and the like. In this case, the concentration of alcohol is not particularly limited, and may be equal to or less than 70%, equal to or less than 60%, equal to or less than 50%, equal to or less than 40%, equal to or less than 30%, equal to or less than 20%, or equal to or less than 10%. However, the concentration of alcohol is preferably equal to or less than 5% or equal to or less than 2%, more preferably equal to or less than 1% or equal to or less than 0.5%, and most preferably equal to or less than 0.2% or equal to or less than 0.1%.

The washing solution plug 45 may be constituted by a plurality of plugs divided by oil plugs. When the washing solution plug 45 is made of a plurality of plugs, the liquids of the respective plugs may be the same or different from one another. If at least one of the plugs is a plug of a washing solution, the liquids of other plugs are not particularly limited. However, it is preferable that all plugs are washing solutions. The number of divided plugs of the washing solution plug 45 can be appropriately set in consideration of, for example, the length of the tube 20 or the subject to be washed.

The elution solution 47 refers to the liquid that causes the nucleic acid adsorbed onto the nucleic acid-binding solid-phase carriers to be eluted in the solution from the carriers to cause a reverse transcription reaction and a polymerase reaction. Therefore, the elution solution 47 may be water or a buffer and may be prepared in advance such that the elution solution 47, in which the nucleic acid has been eluted, is directly used as a buffer solution for use in a reverse transcription reaction and a polymerase reaction. The salt which makes the elution solution be a buffer solution is not particularly limited as long as the salt does not hinder an enzyme reaction, and salts, such as TRIS, HEPES, PIPES, and phosphoric acid, are preferably used. For a reverse transcription reaction, the elution solution 47 may contain a reverse transcriptase, dNTP, and a primer for a reverse transcriptase (oligonucleotide). It is preferable that the elution solution 47 contains bovine serum albumin (BSA) or gelatin as a reaction hindrance inhibitor. The solvent is preferably water and more preferably an organic solvent, such as ethanol or isopropyl alcohol and a solvent which does not substantially contain a chaotropic substance.

A primer for DNA polymerase can easily determine an appropriate sequence for DNA to be detected. Generally, in order to amplify one type of DNA, a primer pair of a 5' side primer and a 3' side primer may be contained. In order to amplify a plurality of types of DNA, if a plurality of primer pairs labeled with different fluorescent dyes are contained, it is possible to cope with multiplex PCR. In this case, a plurality of TaqMan probes may be appropriately provided.

The concentration of dNTP or salt contained in the elution solution may be appropriately set depending on the enzyme to be used. Generally, the concentration of dNTP is 10 to 1000 µM and preferably 100 to 500 µM, the concentration of Mg²⁺ is 1 to 100 mM and preferably 5 to 10 mM, and the concentration of Cl⁻ is 1 to 2000 mM and preferably 200 to 700 mM. The total ion concentration is not particularly limited, and is greater than 50 mM, preferably greater than 100 mM, more preferably greater than 120 mM, still more preferably greater than 150 mM, and even more preferably greater than 200 mM. The upper limit thereof is preferably equal to or less than 500 mM, more preferably equal to or less than 300 mM, and still more preferably equal to or less than 200 mM. The oligonucleotides for a primer are used at a concentration of 0.1 to 10 µM and preferably 0.1 to 1 µM. If the concentration of BSA or gelatin is equal to or less than 1 mg/mL, the reaction hindrance inhibitory effect is diminished, and if the concentration of BSA or gelatin is equal to or greater than 10 mg/mL, the reverse transcription reaction or the following enzyme reaction may be hindered. Therefore, the concentration is preferably 1 to 10 mg/mL. When gelatin is used, the source thereof is not particularly limited, and examples thereof include cowhide, pig hide, and beef bones. When gelatin is not easily dissolved, gelatin may be dissolved by heating.

The volume of the elution solution plug 47 is not particularly limited and can be appropriately set based on an index, such as the amount of particles and the like onto which nucleic acid is adsorbed. For example, when the volume of the particles and the like is 0.5 µL, it should suffice that the volume of the elution solution plug 47 is equal to or greater than 0.5 µL. The volume is preferably equal to or greater than 0.8 µL and equal to or less than 5 µL, and more preferably equal to or greater than 1 µL and equal to or less than 3 µL. If the volume of the elution solution plug 47 is within the above range, it is possible to cause the nucleic acid to be sufficiently eluted from the carriers, for example, even if the volume of the nucleic acid-binding solid-phase carriers is set to 0.5 µL.

The downstream portion of the capillary 23 is inserted into the PCR container 30. As a result, if the elution solution plug 47 in the tube 20 is pushed out from the tube 20, the elution solution 47 can be introduced into the PCR container 30.

A cyclic convexity of the outer wall of the capillary 23 comes into contact with the inner wall of the PCR container 30, whereby an upper seal portion is formed. The outer wall of the capillary 23 on the downstream side from the upper seal portion comes into contact with the inner wall of the PCR container 30, whereby a lower seal portion is formed. The upper seal portion and the lower seal portion will be described below.

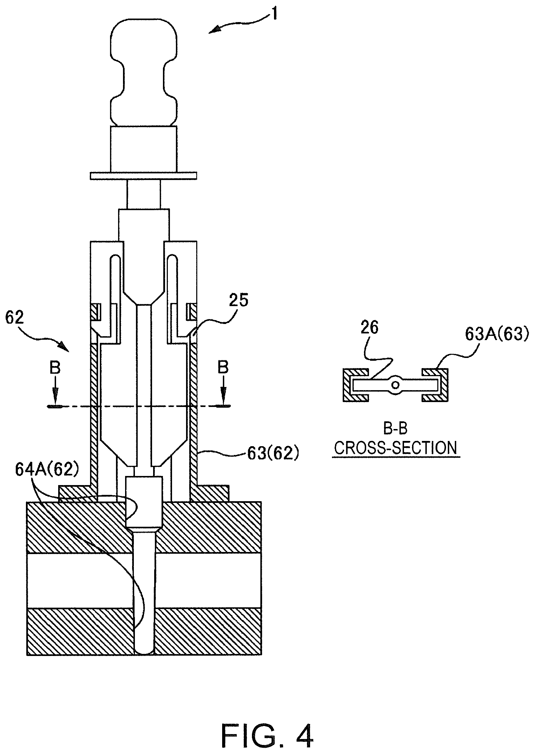

The tube 20 further has the fixing claw 25 and the guide plate 26. Fig. 4 is a diagram illustrating the fixing claw 25, the guide plate 26, and the mounting portion 62.

The fixing claw 25 is a member for fixing the cartridge 1 to the mounting portion 62. When the cartridge 1 is inserted into the mounting portion 62 until the fixing claw 25 is hooked on, the cartridge 1 is fixed to a normal position of the mounting portion 62. In other words, when the cartridge 1 is in an abnormal position of the mounting portion 62, the fixing claw 25 is not hooked on the mounting portion 62.

The guide plate 26 is a member for guiding the cartridge 1 when the cartridge 1 is mounted on the mounting portion 62 of the PCR device 50. A guide rail 63A is formed in the mounting portion 62 of the PCR device 50. The cartridge 1 is inserted into and fixed to the mounting portion 62 while being guided along the guide rail 63A by the guide plate 26 of the tube 20. The cartridge 1 is long. However, since the cartridge 1 is inserted into the mounting portion 62 under the guidance of the guide plate 26, it is easy to fix the cartridge 1 to a normal position of the mounting portion 62.

The fixing claw 25 and the guide plate 26 are plate-like members protruding from the right and left of the capillary 23. When the magnetic particles 7 in the tube 20 are moved by using a magnet, the magnet approaches the tube from the direction perpendicular to the plate-like fixing claw 25 or the guide plate 26. With this, the distance between the magnet and the magnetic particles 7 in the tube 20 can be shortened. However, the fixing claw 25 and the guide plate 26 may have other shapes as long as the distance between the magnet and the magnetic particles 7 in the tube 20 is shortened.

### 2-3. PCR Container

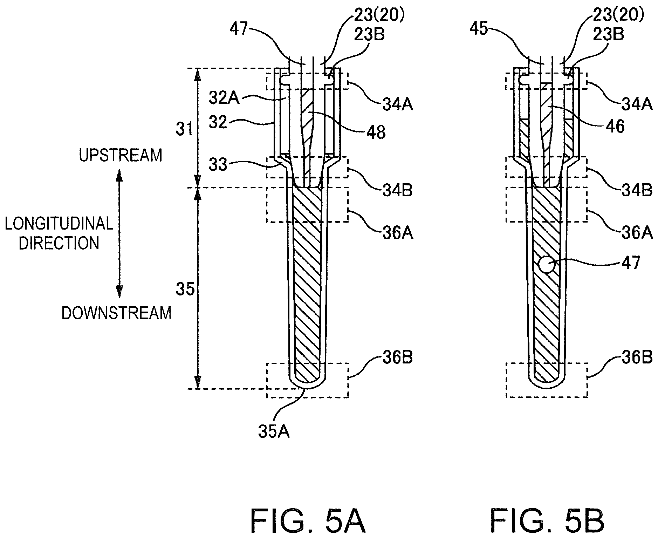

Figs. 5A and 5B are diagrams illustrating the periphery of the PCR container 30. Fig. 5A is a diagram illustrating the initial state. Fig. 5B is a diagram illustrating the state after the plunger 10 is pushed. Hereinafter, the PCR container 30 will be described also referring to Figs. 2A to 2C.

The PCR container 30 is a container which receives the liquid pushed out from the tube 20 and accommodates the elution solution 47 during the thermal cycle process. The PCR container corresponds to a nucleic acid amplification reaction container.

The PCR container 30 has a seal forming portion 31 and a flow path forming portion 35. The seal forming portion 31 is a portion into which the tube 20 is inserted and which inhibits the oil, which overflows from the flow path forming portion 35, from leaking to the outside. The flow path forming portion 35 is placed on the downstream side from the seal forming portion 31 and forms a flow path through which the elution solution 47 in the form of a droplet moves. The PCR container 30 is fixed to the tube 20 at two sites including an upper seal portion 34A and a lower seal portion 34B of the seal forming portion 31.

The seal forming portion 31 has an oil accommodating portion 32 and a stepped portion 33.

The oil accommodating portion 32 is a cylindrical portion and functions as a reservoir accommodating the oil which overflows from the flow path forming portion 35. There is a gap between the inner wall of the oil accommodating portion 32 and the outer wall of the capillary 23 of the tube 20, and this gap becomes an oil accommodating space 32A accommodating the oil which overflows from the flow path forming portion 35. The volume of the oil accommodating space 32A is greater than the volume in which the seal 12A of the plunger 10 slides in the lower syringe 22 of the tube 20.

The inner wall on the upstream side of the oil accommodating portion 32 comes into contact with the cyclic convexity of the tube 20, whereby the upper seal portion 34A is formed. The upper seal portion 34A is a seal which allows permeation of air while inhibiting the oil of the oil accommodating space 32A from leaking to the outside. In the upper seal portion 34A, a vent having such a size that does not allow the oil to leak due to the surface tension of the oil is formed. The vent of the upper seal portion 34A may be a gap between the convexity of the tube 20 and the inner wall of the oil accommodating portion 32, or may be a hole, groove, or notch formed in the convexity of the tube 20. The upper seal portion 34A may be formed of an oil absorbent absorbing oil.

The stepped portion 33 is a portion which is disposed on the downstream side of the oil accommodating portion 32 and shows a step difference. The inner diameter of the downstream portion of the stepped portion 33 is smaller than the inner diameter of the oil accommodating portion 32. The inner wall of the stepped portion 33 comes into contact with the outer wall on the downstream side of the capillary 23 of the tube 20. The inner wall of the stepped portion 33 comes into contact with the outer wall of the tube 20, whereby the lower seal portion 34B is formed. The lower seal portion 34B is a seal which allows the oil of the flow path forming portion 35 to flow to the oil accommodating space 32A while exhibiting resistance to the flow. Due to pressure loss of the lower seal portion 34B, the pressure in the flow path forming portion 35 becomes higher than the outside pressure. Therefore, even if the liquid in the flow path forming portion 35 is heated during the thermal cycle process, air bubbles are not easily formed in the liquid in the flow path forming portion 35.

The flow path forming portion 35 is a tubular portion and functions as a container forming a flow path through which the elution solution 47 in the form of a droplet moves. The flow path forming portion 35 is filled with oil. The upstream side of the flow path forming portion 35 is closed by the terminal of the tube 20, and the terminal of the tube 20 is opened toward the flow path forming portion 35. The inner diameter of the flow path forming portion 35 is greater than the inner diameter of the capillary 23 of the tube 20, and is also greater than the outer diameter of a sphere of the liquid having the volume of the elution solution plug 47 which is formed in a sphere. It is desirable that the inner wall of the flow path forming portion 35 exhibits water repellency to such a degree that the water-soluble elution solution 47 does not adhere to the inner wall.

The upstream side of the flow path forming portion 35 is heated at a relatively high temperature (for example, about 95°C) by the first heater 65A of the outside, thereby forming the high temperature region 36A. The downstream side of the flow path forming portion 35 is heated at a relatively low temperature (for example, about 60°C) by a second heater 65E of the outside, thereby forming the low temperature region 36B. The bottom 35A (edge of the downstream side) of the PCR container 30 includes the low temperature region 36B. As a result, a temperature gradient is formed in the liquid in the flow path forming portion 35.

As shown in Fig. 5A, in the initial state, the flow path forming portion 35 of the PCR container 30 is filled with oil. The interface of the oil is positioned on the comparatively downstream side from the oil accommodating space 32A. In the oil accommodating space 32A, the volume of the upstream side from the interface of the oil is greater than the volume in which the seal 12A of the plunger 10 slides in the lower syringe 22 of the tube 20.

As shown in Fig. 5B, when the plunger 10 is pushed, the liquid in the tube 20 is pushed out into the flow path forming portion 35. Since the liquid in the tube 20 is pushed out into the flow path forming portion 35 which has already been filled with oil, gas does not flow into the flow path forming portion 35.

When the plunger 10 is pushed, first, the third oil plug 48 of the tube 20 flows into the flow path forming portion 35, and the inflow oil then flows into the oil accommodating space 32A from the flow path forming portion 35, whereby the oil interface of the oil accommodating space 32A ascends. At this time, due to pressure loss of the lower seal portion 34B, the pressure of liquid in the flow path forming portion 35 increases. After the third oil plug 48 is pushed out from the tube 20, the elution solution plug 47 flows into the flow path forming portion 35 from the tube 20. Since the inner diameter of the flow path forming portion 35 is greater than the inner diameter of the capillary 23, the elution solution 47, which has been in the form of a plug (a column) in the tube 20, becomes a droplet in the oil in the flow path forming portion 35. In the initial state, the volume of the oil accommodating space 32A which is on the upstream side from the interface of oil is greater than the volume in which the seal 12A of the plunger 10 slides in the lower syringe 22 of the tube 20. Therefore, the oil does not overflow from the oil accommodating space 32A.

### PCR Device 50

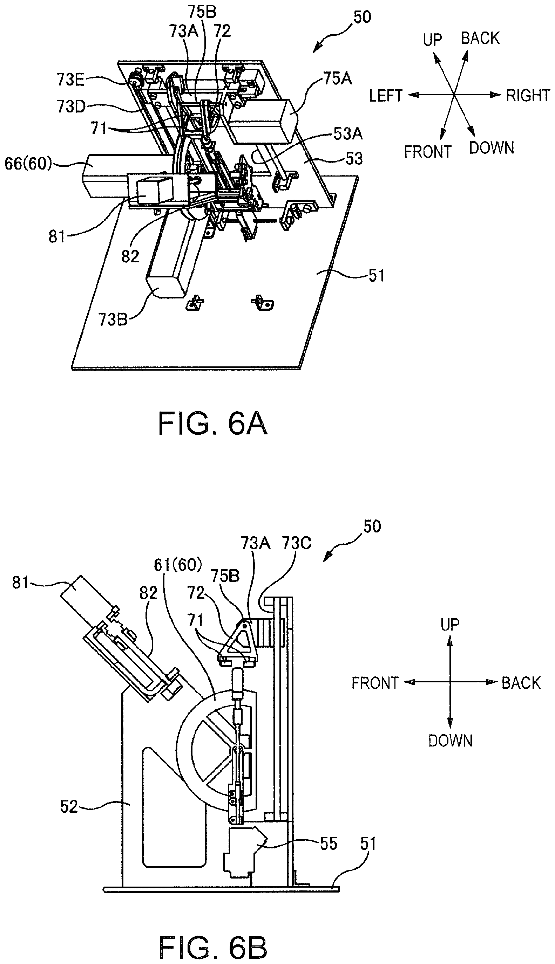

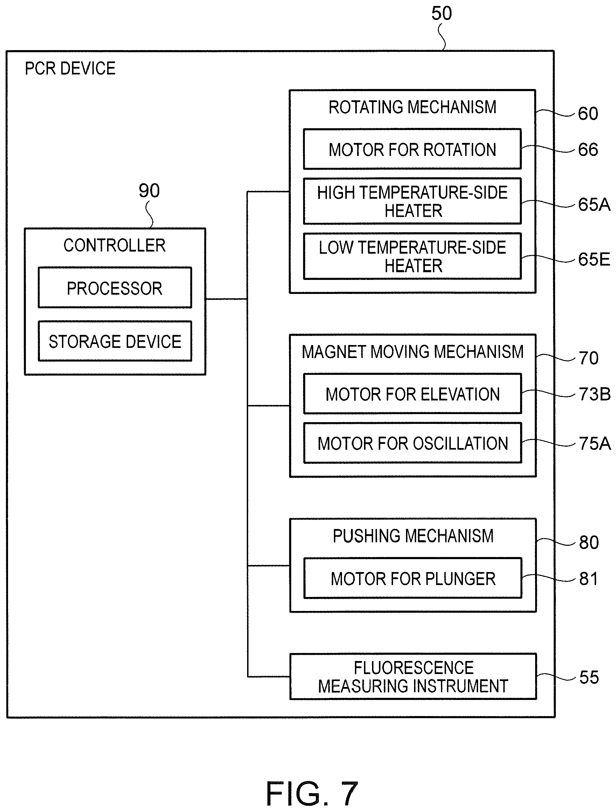

Fig. 6A is a perspective view showing the internal configuration of the PCR device 50. Fig. 6B is a side view showing the main configuration of the PCR device 50. Fig. 7 is a block diagram of the PCR device 50. The PCR device 50 is a device which performs a nucleic acid elution process and a thermal cycle process using the cartridge 1.

In the following description of the PCR device 50, the meanings of "up and down", "front and back", and "left and right" will be defined as shown in the drawing. When a base 51 of the PCR device 50 is horizontally disposed, a direction perpendicular to the base 51 is defined as an "up-down direction", and "up" and "down" will be defined according to the direction of gravity. The axial direction of the rotary shaft of the cartridge 1 is defined as a "right-left direction" , and a direction perpendicular to the up-down direction and the right-left direction is defined as a "front-back direction". When the PCR device 50 is viewed from the rotary shaft of the cartridge 1, a cartridge insertion port 53A side is defined as "back", and a side opposite to "back" is defined as "front". When the PCR device 50 is viewed from the front side, the right side and the left side in the right-left direction are respectively defined as "right" and "left".

The PCR device 50 has a rotating mechanism 60, a magnet moving mechanism 70, a pushing mechanism 80, a fluorescence measuring instrument 55, and a controller 90.

### 1. Rotating Mechanism 60

The rotating mechanism 60 is a mechanism for rotating the cartridge 1 and the heater. When the cartridge 1 and the heater are moved upside down by the rotating mechanism 60, the elution solution 47 in the form of a droplet moves inside the flow path forming portion 35 of the PCR container 30, whereby the thermal cycle process is performed.

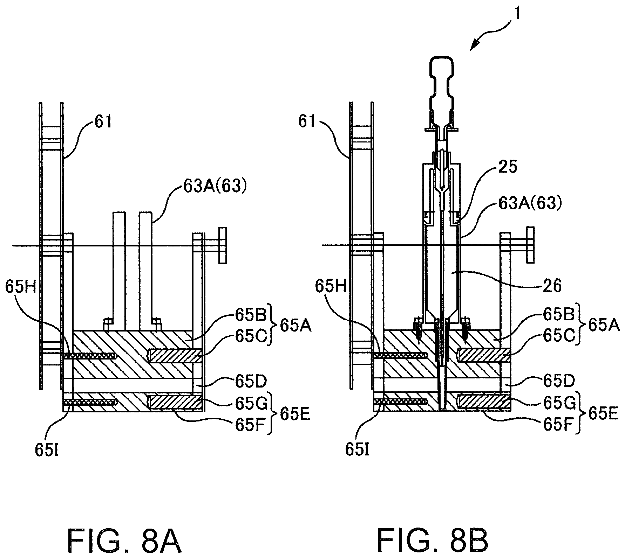

The rotating mechanism 60 has a rotary body 61 and a motor for rotation 66. Fig. 8A is a diagram illustrating the rotary body 61. Fig. 8B is a diagram illustrating the state where the cartridge 1 is mounted on the mounting portion 62 of the rotary body 61.

The rotary body 61 is a member rotatable around the rotary shaft. The rotary shaft of the rotary body 61 is supported on a support 52 fixed to the base 51. The rotary body 61 is provided with the mounting portion 62 on which the cartridge 1 is mounted and the heaters (the first heater 65A and the second heater 65E). If the rotary body 61 rotates, the cartridge 1 can be moved upside down in a state where the positional relationship between the cartridge 1 and the heater is maintained. The motor for rotation 66 is a power source for rotating the rotary body 61. The motor for rotation 66 rotates the rotary body 61 to a predetermined position according to the instruction from the controller 90. A transmission mechanism, such as a gear, may be interposed between the motor for rotation 66 and the rotary body 61.

The rotary shaft of the rotary body 61 is positioned closer to the tube 20 than to the PCR container 30 of the cartridge 1. In other words, the rotary shaft of the rotary body 61 is positioned in the tube 20 of the cartridge 1 mounted on the mounting portion 62. This is because the tube 20 is longer than the PCR container 30, and therefore, if the center of the PCR container 30 is used as the rotary shaft (if the rotary shaft of the rotary body 61 is positioned in the PCR container), the size of the rotary body 61 will increase.

The mounting portion 62 is a portion on which the cartridge 1 is mounted. The mounting portion 62 has a fixing portion 63 in which a notch is formed. An insertion hole 64A formed in the heaters (the first heater 65A and the second heater 65E) also functions as the mounting portion 62. When the fixing claw 25 of the cartridge 1 is hooked on the notch of the fixing portion 63 in a state where the PCR container 30 is inserted into the insertion hole 64A, the cartridge 1 is mounted on the rotary body 61 (see Fig. 4). Here, a portion of the heater also functions as the mounting portion 62, but the mounting portion 62 may be separated from the heater. The mounting portion 62 is indirectly fixed to the rotary body 61 through the first heater 65A. However, the mounting portion 62 may be directly disposed in the rotary body 61. The number of the cartridge 1 that can be mounted on the mounting portion 62 is not limited to one, and a plurality of cartridges 1 may be mounted on the mounting portion 62.

The fixing portion 63 of the mounting portion 62 functions as a tube fixing portion to which the tube 20 of the cartridge 1 is fixed. The insertion hole 64A functions as a PCR container fixing portion to which the PCR container 30 is fixed. With this, the long cartridge 1 including the tube 20 and the PCR container 30 is stably fixed to the mounting portion 62.

The guide rail 63A is formed in the fixing portion 63 along the up-down direction (see Fig. 4). The guide rail 63A guides the guide plate 26 of the cartridge 1 in the insertion direction while constraining the guide plate 26 in the front-back direction. While the guide plate 26 is being guided by the guide rail 63A, the cartridge 1 is inserted into the mounting portion 62. Therefore, the PCR container 30 of the cartridge 1 is guided to the insertion hole 64A, whereby the cartridge 1 is fixed to a normal position of the mounting portion 62.

The PCR device 50 has the first heater 65A and the second heater 65E. Each of the heaters is constituted by a heating source and a heat block. The heating source is, for example, a cartridge heater 65C or 65G and is inserted into the heat block 65B or 65F. The heat blocks 65B and 65F are, for example, a metal with high heat conductivity, such as aluminum, and heat the liquid in the cartridge 1 with heat from the heating sources while suppressing heat unevenness. It is desirable for the heat blocks 65B and 65F to be a nonmagnetic substance to prevent the magnets 71, which move the magnetic particles 7, from being adsorbed onto the heat blocks.

The first heater 65A is a heater which heats the elution solution plug 47 of the cartridge 1 and the upstream side of the flow path forming portion 35 of the PCR container 30. If the cartridge 1 is fixed to a normal position, the first heater 65A faces the elution solution plug 47 of the tube 20 and the upstream side (high temperature region 36A) of the flow path forming portion 35 of the PCR container 30. For example, if the first heater 65A heats the elution solution plug 47 to about 65°C, the liberation of the nucleic acid from the magnetic particles is accelerated. When the thermal cycle process is performed, for example, the liquid on the upstream side of the flow path forming portion 35 of the PCR container 30 is heated to about 90°C to 100°C.

The second heater 65E is a heater which heats the bottom 35A of the flow path forming portion 35 of the PCR container 30. If the cartridge 1 is fixed to the normal position, the second heater 65E faces the downstream side (low temperature region 36B) of the flow path forming portion 35 of the PCR container 30. For example, the second heater 65E heats the liquid in the low temperature region 36B of the PCR container 30 to about 50°C to 75°C.

A spacer 65D is disposed between the first heater 65A and the second heater 65E. The spacer 65D suppresses heat conduction between the first heater 65A and the second heater 65E. The spacer 65D is also used for accurately setting the distance between the first heater 65A and the second heater 65E. With this, a temperature gradient is formed in the liquid in the flow path forming portion 35 of the PCR container 30 by the first heater 65A and the second heater 65E.

In the heat block constituting each of the first heater 65A and the second heater 65E, a through hole constituting the insertion hole 64A is formed. The outer wall of the bottom 35A of the PCR container 30 is exposed through the lower opening of the insertion hole 64A of the second heater 65E. The fluorescence measuring instrument 55 measures the luminance of the elution solution 47 from the lower opening of the insertion hole 64A.

Each of the first heater 65A and the second heater 65E is provided with a temperature control device. Accordingly, the temperature can be set to a level suitable for each polymerase reaction.

### 2. Magnet Moving Mechanism 70

The magnet moving mechanism 70 is a mechanism moving the magnets 71. The magnet moving mechanism 70 causes the magnetic particles 7 in the cartridge 1 to be attracted to the magnets 71 and moves the magnets 71 such that the magnetic particles 7 move to the inside of the cartridge 1. The magnet moving mechanism 70 has a pair of magnets 71, an elevating mechanism 73, and an oscillating mechanism 75.

The magnets 71 are members attracting the magnetic particles 7. As the magnets 71, a permanent magnet, an electromagnet, and the like can be used. Here, a permanent magnet that does not generate heat or the like is used. The pair of magnets 71 is held in an arm 72 so as to face each other in the front-back direction in a state where the position thereof in the up-down direction is substantially the same. The respective magnets 71 can face each other on the front side or the back side of the cartridge 1 mounted on the mounting portion 62. The pair of magnets 71 can sandwich the cartridge 1 mounted on the mounting portion 62 therebetween in the front-back direction. If the magnets 71 face each other in a direction (herein, front-back direction) orthogonal to the direction (herein, right-left direction) in which the fixing claw 25 or the guide plate 26 of the cartridge 1 is disposed, the distance between the magnetic particles 7 in the cartridge 1 and the magnets 71 can be shortened.

The elevating mechanism 73 is a mechanism for moving the magnets 71 in the up-down direction. Since the magnets 71 attract the magnetic particles 7, if the magnets 71 are moved in the up-down direction according to the movement of the magnetic particles 7, the magnetic particles 7 in the cartridge 1 can be attracted in the up-down direction.

The elevating mechanism 73 has a carriage 73A moving in the up-down direction and a motor for elevation 73B. The carriage 73A is a member movable in the up-down direction and is guided so as to be movable in the up-down direction by a carriage guide 73C disposed in a sidewall 53 where the cartridge insertion port 53A is placed. The arm 72 holding a pair of magnets 71 is attached to the carriage 73A. Therefore, if the carriage 73A moves in the up-down direction, the magnets 71 move in the up-down direction. The motor for elevation 73B is a power source for moving the carriage 73A in the up-down direction. The motor for elevation 73B moves the carriage 73A to a predetermined position in the up-down direction according to the instruction from the controller 90. Although the motor for elevation 73B moves the carriage 73A in the up-down direction using a belt 73D and a pulley 73E, the motor for elevation 73B may move the carriage 73A in the up-down direction by other transmission mechanisms.

When the carriage 73A is at the uppermost position (retraction position), the magnets 71 are positioned above the cartridge 1. When the carriage 73A is at the retraction position, the elevating mechanism 73 does not come into contact with the cartridge 1 even if the cartridge 1 rotates. The elevating mechanism 73 can bring down the carriage 73A to a position where the magnets 71 face the reaction plug. Therefore, the elevating mechanism 73 can move the magnets 71 such that the magnetic particles 7 in the tank 3 move to the position of the reaction plug.

The oscillating mechanism 75 is a mechanism for oscillating the pair of magnets 71 in the front-back direction. When the pair of magnets 71 oscillates in the front-back direction, each of the magnets 71 becomes distant from the cartridge 1 by a different length. Since the magnetic particles 7 are attracted to the magnets 71 close to the cartridge 1, if the pair of magnets 71 oscillates in the front-back direction, the magnetic particles 7 in the cartridge 1 move in the front-back direction.

The oscillating mechanism 75 has a motor for oscillation 75A and a gear. The motor for oscillation 75A and the gear are disposed in the carriage 73A and can move in the up-down direction together with the carriage 73A. When the power of the motor for oscillation 75A is transmitted to the arm 72 through the gear, the arm 72 holding the magnets 71 rotates around an oscillating rotary shaft 75B relative to the carriage 73A. In order to prevent damage to the cartridge 1 due to contact between the magnets 71 and the cartridge 1, the oscillating mechanism 75 causes the magnets 71 to oscillate within a range in which the magnets 71 do not come into contact with the cartridge 1.

The oscillating rotary shaft 75B is a rotary shaft of the arm 72. The oscillation rotary shaft 75B is in parallel with the horizontal direction so as to cause the magnets 71 to oscillate in the front-back direction. When the oscillating rotary shaft 75B is viewed from the right or left, the oscillating rotary shaft 75B is disposed at a position depart from the cartridge 1 toward the front or back of the cartridge. Therefore, when the carriage 73A moves to the lower side, the contact between the cartridge 1 and the arm 72 can be avoided. If the magnets 71 can oscillate in the front-back direction, the oscillating rotary shaft 75B may be a shaft in parallel with the up-down direction.

### 3. Pushing Mechanism 80

The pushing mechanism 80 is a mechanism for pushing the plunger 10 of the cartridge 1. When the plunger 10 is pushed by the pushing mechanism 80, the elution solution plug 47 and oil plug of the cartridge 1 are pushed out to the PCR container 30, whereby the elution solution 47 in the form of a droplet is formed in the oil of the PCR container 30.

The pushing mechanism 80 has a motor for a plunger 81 and a rod 82. The motor for a plunger 81 is a source of power for moving the rod 82. The rod 82 is a member for pushing the mounting board 11A of the plunger 10 of the cartridge 1. The reason for pushing the mounting board 11A instead of the tank 3 of the cartridge 1 is that the tank 3 is made of flexible resin which is dilatable. When the tank 3 is not deformed, the pushing mechanism 80 may push the tank 3 to push the plunger 10.

The plunger 10 is pushed by the rod 82, not in the up-down direction but in a direction inclined from the up-down direction by 45 degrees. Accordingly, when the plunger 10 is pushed by the pushing mechanism 80, in the PCR device 50, the rotary body 61 is rotated by 45 degrees to match the longitudinal direction of the cartridge 1 with the movement direction of the rod 82, and then the rod 82 is moved. Since the rod 82 pushes the plunger 10 in the direction inclined from the up-down direction by 45 degrees, it is easy to dispose the pushing mechanism 80 such that this mechanism becomes free from interference of the elevating mechanism 73. Since the rod 82 pushes the plunger 10 in the direction inclined from the up-down direction by 45 degrees, the size of the PCR device 50 can be reduced in the up-down direction.

### 4. Fluorescence Measuring Instrument 55

The fluorescence measuring instrument 55 is a measuring instrument which measures luminance of the elution solution 47 of the PCR container 30. The fluorescence measuring instrument 55 is disposed below the rotary body 61 so as to face the bottom 35A of the PCR container 30 of the cartridge 1. The fluorescence measuring instrument 55 measure luminance of the elution solution 47 at the bottom 35A of the PCR container 30 from the lower opening of the insertion hole 64A of the second heater 65E. The fluorescence measuring instrument 55 can detect luminance of a plurality of wavelength regions so as to cope with multiplex PCR.

### 5. Controller 90

The controller 90 is a control unit which controls the PCR device 50. The controller 90 has, for example, a processor, such as CPU, and a storage device, such as a ROM or a RAM. The stored device stores various programs and data. The storage device provides an area for running the programs. When the processor executes a program stored in the storage device, various processes are performed.

For example, the controller 90 controls the motor for rotation 66 to rotate the rotary body 61 to a predetermined rotation position. The rotating mechanism 60 is provided with a rotation position sensor (not shown), and the controller 90 drives and stops the motor for rotation 66 according to a detection result of the rotation position sensor.

The controller 90 controls the heaters (the first heater 65A and the second heater 65E) such that each heater generates heat. The heat blocks constituting the heaters are respectively provided with temperature sensors 65H and 65I and the controller 90 controls ON and OFF of the cartridge heaters according to detection results of the temperature sensors 65H and 65I.

The controller 90 controls the motor for elevation 73B to move the magnets 71 in the up-down direction. The PCR device 50 is provided with a position sensor (not shown) which detects the position of the carriage 73A, and the controller 90 drives and stops the motor for elevation 73B according to a detection result of the position sensor.

The controller 90 controls the motor for oscillation 75A to oscillate the magnet 71 in the front-back direction. The PCR device 50 is provided with a position sensor which detects the position of the arm 72 holding the magnets 71, and the controller 90 drives and stops the motor for oscillation 75A according to a detection result of the position sensor.

The controller 90 controls the fluorescence measuring instrument 55 to measure luminance of the elution solution 47 of the PCR container 30. The controller 90 causes the fluorescence measuring instrument 55 to perform measurement when the fluorescence measuring instrument 55 faces the bottom 35A of the PCR container 30 of the cartridge 1. A measurement result is stored in the storage device.

### Description of Operation

### 1. Operation for Mounting Cartridge 1

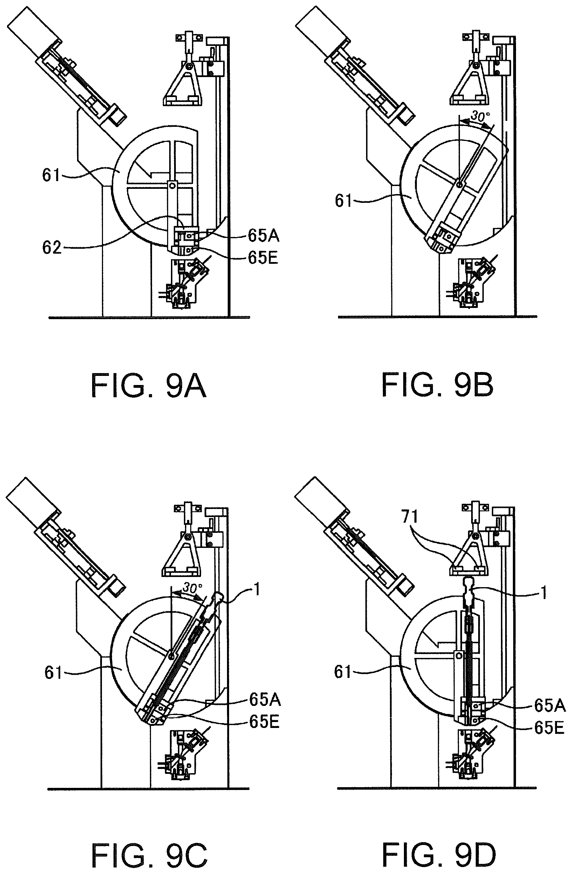

Figs. 9A to 9D are diagrams illustrating the state of the PCR device 50 when mounting the cartridge 1. Fig. 9A is a diagram illustrating an initial state where the cartridge 1 is mounted. Fig. 9B is a diagram illustrating a standby state. Fig. 9C is a diagram illustrating the state immediately after the cartridge 1 is mounted. Fig. 9D is a diagram illustrating an initial state in the state where the cartridge 1 is mounted.

As shown in Fig. 9A, in the initial state before the cartridge 1 is mounted, the mounting direction of the mounting portion 62 is in the up-down direction. In the following description, the rotation position of the rotary body 61 in this state is taken as a reference (0 degrees), and if the rotary body 61 rotates in a counter clockwise direction when viewed from the right, the direction is referred to as a positive direction.

As shown in Fig. 9B, the controller 90 drives the motor for rotation 66 to rotate the rotary body 61 by -30°. In this state, an operator inserts the cartridge 1 into the mounting portion 62 from the cartridge insertion port 53A. At this time, since the cartridge 1 is inserted into the mounting portion 62 while the guide plate 26 is being guided by the guide rail 63A, the PCR container 30 of the cartridge 1 is guided to the insertion hole 64A of the mounting portion 62. The operator inserts the cartridge 1 until the fixing claw 25 of the cartridge 1 is hooked on the notch of the fixing portion 63. With this, the cartridge 1 is fixed to a normal position of the mounting portion 62. When the PCR container 30 is not inserted into the insertion hole 64A, and the cartridge 1 is fixed to an abnormal position of the mounting portion 62, the fixing claw 25 of the cartridge 1 is not hooked on the notch of the fixing portion 63. Accordingly, the operator can recognize that the cartridge 1 is at the abnormal position.

As shown in Fig. 9C, when the cartridge 1 is fixed to a normal position of the mounting portion 62, the elution solution plug 47 of the tube 20 and the upstream side (high temperature region 36A) of the flow path forming portion 35 of the PCR container 30 face the first heater 65A, and the downstream side (low temperature region 36B) of the flow path forming portion 35 of the PCR container 30 faces the second heater 65C. Since the mounting portion 62 and the heaters are disposed in the rotary body 61, even when the rotary body 61 rotates, the positional relationship between the cartridge 1 and the heaters is maintained in this state.

After the cartridge 1 is mounted on the mounting portion 62, as shown in Fig. 9D, the controller 90 controls the rotary body 61 to rotate by 30 degrees such that the rotary body 61 returns to the reference. The controller 90 may detect the state where the cartridge 1 is mounted on the mounting portion 62 using a sensor (not shown). Alternatively, the controller 90 may detect the state by an operator's input operation.

### 2. Nucleic Acid Elution Treatment

### Up-and-Down Motion of Magnets 71

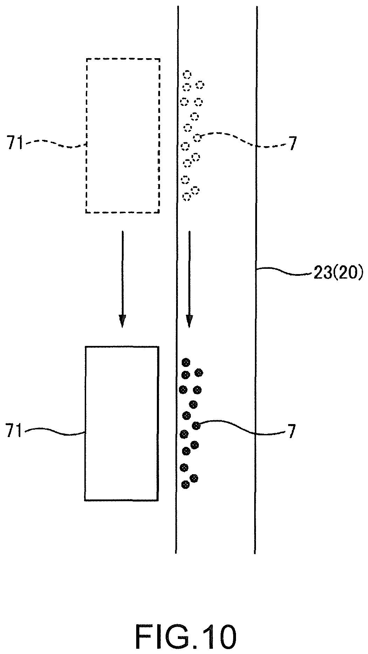

Fig. 10 is a conceptual diagram of the behavior of the magnetic particles 7 when the magnet 71 is moved downward. The magnetic particles 7 in the cartridge 1 are attracted to the magnet 71. For this reason, if the magnet 71 moves outside the cartridge 1, the magnetic particles 7 in the cartridge 1 move along with the magnet 71.

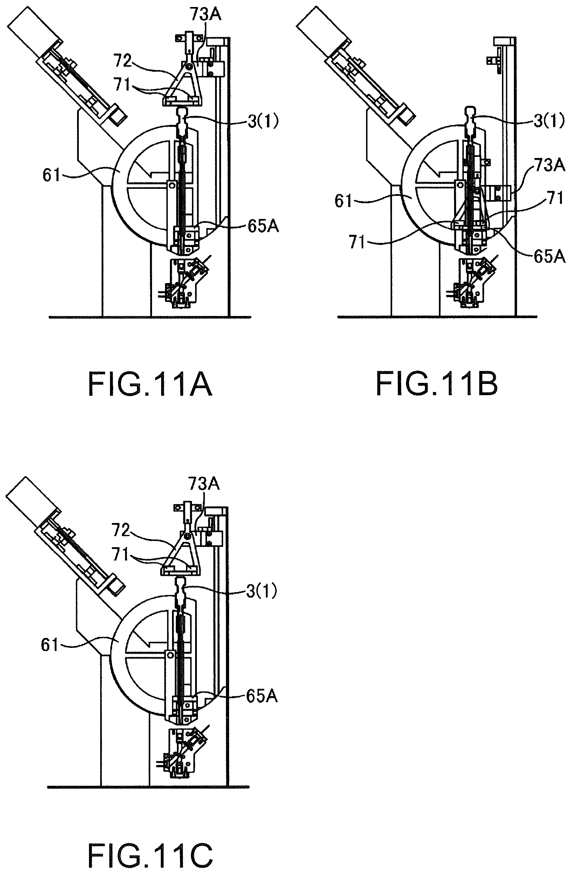

Figs. 11A to 11C are diagrams illustrating a nucleic acid elution process. Fig. 11A is a diagram illustrating the state of the PCR device 50 before the nucleic acid elution process. Fig. 11B is a diagram illustrating the state of the PCR device 50 when the magnets 71 are moved to the elution solution plug 47. Fig. 11C is a diagram illustrating the state of the PCR device 50 when the magnets 71 are drawn up.

As shown in Fig. 11A, in the initial state, the tank 3 becomes the upper side of the cartridge 1, and the longitudinal direction of the cartridge 1 is in parallel with the vertical direction. In this state, as shown in Fig. 2A, the cartridge 1 has the dissolution solution 41 (tank 3) containing the magnetic particles 7, the oil 42 (plunger 10), the washing solution 43 (upstream side of the tube 20), the first oil plug 44 (capillary 23), the washing solution plug 45 (capillary 23), the second oil plug 46 (capillary 23), the elution solution plug 47 (capillary 23), the third oil plug 48 (capillary 23), and oil (PCR container 30) in this order from the upper side of the cartridge.

As shown in Fig. 11A, in the initial state, the carriage 73A is at the uppermost position (retractionposition), and the magnets 71 are placed above the cartridge 1. In this state, the controller 90 drives the motor for elevation 73B to slowly move downward the carriage 73A such that the magnets 71 slowly move downward. Since the longitudinal direction of the cartridge 1 is in parallel with the vertical direction, the magnets 71 move along the cartridge 1.

When the magnets 71 move downward, the magnets 71 face the tank 3, and the magnetic particles 7 in the tank 3 are attracted to the magnets 71. The controller 90 moves downward the carriage 73A at such a speed that the magnetic particles 7 can move together with the magnets 71.

When the magnets 71 move from the position (the height of the tank 3) facing the tank 3 to the position (the height of the plunger 10) facing the plunger 10, the magnetic particles 7 pass through the opening on the upstream side of the cylindrical portion 11 of the plunger 10 and then pass through the interface between the dissolution solution 41 in the tank 3 and the oil 42 on the upstream side of the cartridge main body 9. With this, the magnetic particles 7 bound to the nucleic acid are introduced into the cartridge main body 9. When the magnetic particles 7 pass through the interface between the dissolution solution 41 and the oil 42, the dissolution solution 41 is wiped by the oil 42. Therefore, the components of the dissolution solution 41 are not easily brought into the oil 42. With this, it is possible to prevent the components of the dissolution solution 41 from being brought into the washing solution or the elution solution 47.

When the magnets 71 move downward in the state of facing the plunger 10, the magnetic particles 7 pass through the inside of the cylindrical portion 11, escape from the inside and outside of the ribs 13, come out from the opening of the downstream side of the cylindrical portion 11, and are introduced into the upper syringe 21 of the tube 20. In the meantime, the magnetic particles 7 pass through the interface between the oil 42 and the washing solution 43, in the plunger 10. When the magnetic particles 7 are introduced into the washing solution 43, the nucleic acid having been bound to the magnetic particles 7 is washed with the washing solution 43.

At this stage, the rod-like portion 12 of the plunger 10 is not yet inserted into the lower syringe 22 of the tube 20. Therefore, when the magnets 71 move from the position (the height of the upper syringe 21) facing the upper syringe 21 to the position (the height of the capillary 23) facing the capillary 23, the magnetic particles 7 move to the lower syringe 22 from the upper syringe 21 and then move to the capillary 23 from the lower syringe 22. The first oil plug 44 is on the upstream side of the capillary 23, and when the magnetic particles 7 move to the capillary 23 from the lower syringe 22, the magnetic particles 7 pass through the interface between the washing solution 43 and the oil. At this time, since the washing solution 43 is wiped by the oil, the components of the washing solution 43 are not easily brought into the oil. In this way, it is possible to prevent the components of the washing solution 43 from being mixed into the washing solution plug 45 or the elution solution plug 47.

If the magnets 71 move from the position (the height of the first oil plug 44) facing the first oil plug 44 to the position (the height of the washing solution plug 45) facing the washing solution plug 45, the magnetic particles 7 pass through the interface between the oil and the washing solution. When the magnetic particles 7 are introduced into the washing solution plug 45, the nucleic acid having been bound to the magnetic particles 7 is washed with the washing solution.

When the magnets 71 move from the position (the height section of the washing solution plug 45) facing the washing solution plug 45 to the position (the height of the second oil plug 46) facing the second oil plug 46, the washing solution and the magnetic particles 7 pass through the interface between the washing solution and the oil. At this time, since the washing solution is wiped by the oil, the components of the washing solution are not easily brought into the oil. With this, it is possible to prevent the components of the washing solution from being mixed into the elution solution plug 47.

When the magnets 71 move from the position (the height of the second oil plug 46) facing the second oil plug 46 to the position (the height of the elution solution plug 47) facing the elution solution plug 47, the magnetic particles 7 pass through the interface between the oil and the elution solution 47.

The controller 90 controls the first heater 65A to heat the elution solution plug 47 before the magnetic particles 7 are introduced into the elution solution plug 47. The heating temperature may be higher than 40°C, preferably equal to or higher than 50°C, and more preferably equal to or higher than 60°C. The upper limit of the heating temperature is not particularly limited, but is preferably equal to or lower than 75°C, more preferably equal to or lower than 70°C, and most preferably 65°C. If the elution solution 47 is heated before the magnetic particles 7 are introduced, the time from when the magnetic particles 7 are introduced into the elution solution 47 until the elution of the nucleic acid is completed can be shortened.

As shown in Fig. 11B, after the magnets 71 move to the position (the height of the elution solution plug 47) facing the elution solution plug 47, the controller 90 stops the motor for elevation 73B to stop the movement of the magnets 71 in the up-down direction and performs the process for 30 seconds, for example, at 65°C. Therefore, the nucleic acid bound to the magnetic particles 7 is liberated into the solution of the elution solution plug 47, and a reverse transcription reaction proceeds. If the elution solution 47 is heated, the elution of nucleic acid from the magnetic particles 7 and the reverse transcription reaction are accelerated.

After the nucleic acid is eluted in the elution solution plug 47, the controller 90 drives the motor for elevation 73B in an opposite direction such that the carriage 73A slowly moves upward to slowly move the magnets 71 upward. The controller 90 moves the carriage 73A upward at such a speed that the magnetic particles 7 can move together with the magnets 71.

If the magnets 71 move upward from the state shown in Fig. 11B, the magnetic particles 7 move from the elution solution plug 47 to the second oil plug 46, whereby the magnetic particles 7 are removed from the elution solution plug 47. It is preferable to move the magnetic particles 7 from the elution solution plug 47 toward the first oil plug 44. For example, the magnetic particles 7 may be moved from the elution solution plug 47 to the first oil plug 44 or the washing solution plug 45.

If the magnets 71 slowly move to the position facing the upper syringe 21, the magnetic particles 7 also move to the upper syringe 21 and are positioned above the lower syringe 22. If the magnetic particles 7 are moved to this position, the magnetic particles 7 are not introduced into the PCR container 30 when the plunger 10 is pushed. For this reason, while the state is changed to the state shown in Fig. 11C from the state described above, the controller 90 may move the carriage 73A upward at such a speed that the magnetic particles 7 are inhibited from following the movement of the magnets 71. In addition, if the magnetic particles 7 are not introduced into the PCR container 30 when the plunger 10 is pushed, the movement speed of the carriage 73A may be increased at an earlier stage.

The storage device of the controller 90 stores information regarding the movement speed of the magnets 71. The controller 90 executes the above operation (operation for moving up and down the magnets 71) according to information.

### Oscillation of Magnets 71

While the magnets 71 are moved vertically, the controller 90 may drive the motor for oscillation 75A such that the pair of magnets 71 with the cartridge 1 sandwiched therebetween oscillates in the front-back direction.

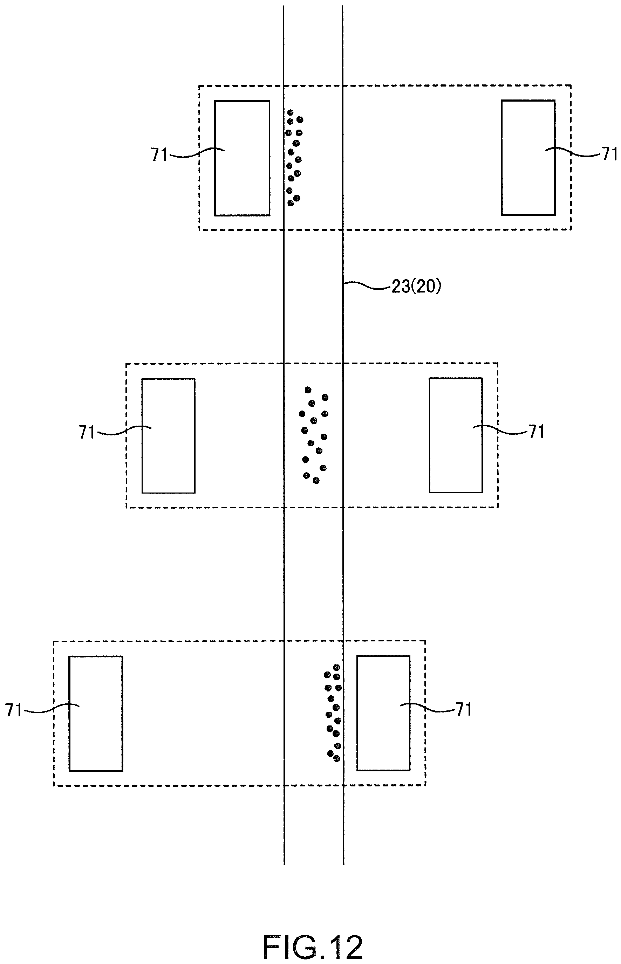

Fig. 12 is a conceptual diagram showing the behavior of the magnetic particles 7 when the magnets 71 oscillate.

While the magnets 71 are moving in the vertical direction, the tube 20 is interposed between the pair of magnets 71 in the front-back direction. Since the pair of magnets 71 is held by the arm 72, the distance between the pair of magnets 71 in the front-back direction is substantially constant. Therefore, one of the pair of magnets 71 approaches the tube 20, and the other magnet is separated from the tube 20.

The magnetic particles 7 are attracted to the magnet 71 close to the magnetic particles. Therefore, when one of the pair of magnets 71 approaches the tube 20, the magnetic particles 7 are attracted to the magnet 71. Thereafter, when the magnet 71 is separated from the tube 20, and then the other magnet 71 approaches the tube 20, the magnetic particles 7 are attracted to the magnet 71. In this way, the magnetic particles 7 move in the front-back direction. When the pair of magnets 71 oscillates in the front-back direction, the magnetic particles 7 reciprocate in the front-back direction.

When the magnetic particles 7 reciprocate in the front-back direction, the magnetic particles 7 easily come into contact with a liquid. Particularly, since the liquid in the capillary 23 hardly exhibits fluidity, when it is desired to make the liquid in the capillary 23 become as close to the magnetic particles 7 as possible, it is effective to cause the magnetic particles 7 to reciprocate in the front-back direction.

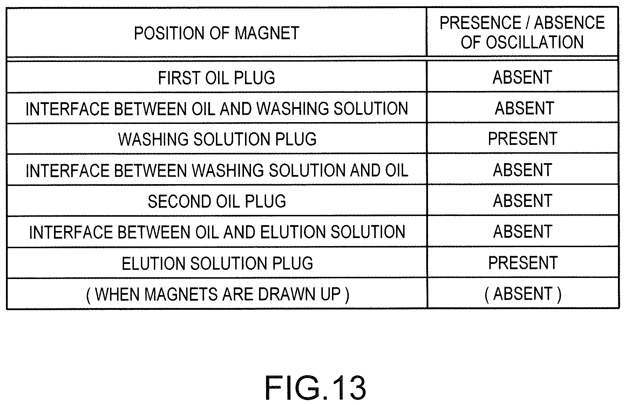

Fig. 13 is a table showing the presence/absence of oscillation of the magnets 71.

When the magnetic particles 7 move the oil plug (the first oil plug 44 or the second oil plug 46) downward, the controller 90 stops the motor for oscillation such that the magnets 71 do not oscillate. At this time, the controller 90 moves the magnets 71 downward in a state where one of the pair of magnets 71 is brought close to the tube 20. This is because the magnetic particles 7 more easily follow the movement of the magnets 71 compared to the case where the magnets 71 are separated from the tube 20 by the same distance.

When the magnetic particles 7 move the washing solution plug 45 downward, the controller 90 drives the motor for oscillation to oscillate the magnets 71 in the front-back direction. With this, the magnetic particles 7 move downward while oscillating in the front-back direction inside the washing solution plug 45. Therefore, the washing effect of the magnetic particles 7 can be enhanced. Since the washing effect is enhanced, the amount of the washing solution plug 45 can be reduced, and the cartridge 1 can be reduced in size.

When the magnetic particles 7 pass through the interface between the washing solution and oil (second oil plug 46), the controller 90 stops the motor for oscillation such that the magnets 71 do not oscillate. With this, since the magnetic particles 7 do not oscillate when passing through the interface, the components of the washing solution are not easily brought into the oil. The controller 90 moves the magnets 71 downward in a state where one of the pair of magnets 71 is brought close to the tube 20. Therefore, the magnetic particles 7 close to the magnet 71 are attracted to the magnet and are aggregated, and the washing solution adhered to the magnetic particles 7 is squeezed out. Therefore, the components of the washing solution are not easily brought into the oil.

When the magnetic particles 7 are in the elution solution plug 47, the controller 90 drives the motor for oscillation such that the magnets 71 oscillate in the front-back direction. With this, since the magnetic particles 7 oscillate in the front-back direction inside the elution solution plug 47, the elution effect of the nucleic acid having been bound to the magnetic particles 7 can be enhanced. Since the elution effect is enhanced, the time from when the magnetic particles 7 are introduced into the elution solution 47 to when the elution of the nucleic acid is completed can be shortened.

When the nucleic acid is eluted in the elution solution plug 47, and then the magnets 71 are moved upward to draw up the magnetic particles 7, the controller 90 stops the motor for oscillation such that the magnets 71 do not oscillate. At this time, the controller 90 moves the magnets 71 downward in a state where one of the pair of magnets 71 is brought close to the tube 20. With this, the magnetic particles 7 easily follow the movement of the magnets 71, and the movement speed of the magnets 71 can be increased.

The storage device of the controller 90 stores information regarding the position of each plug of the capillary 23 and information regarding oscillation as shown in Fig. 13. The controller 90 executes the above operation (operation for oscillating the magnets 71) according to information.

### 3. Droplet Forming Process

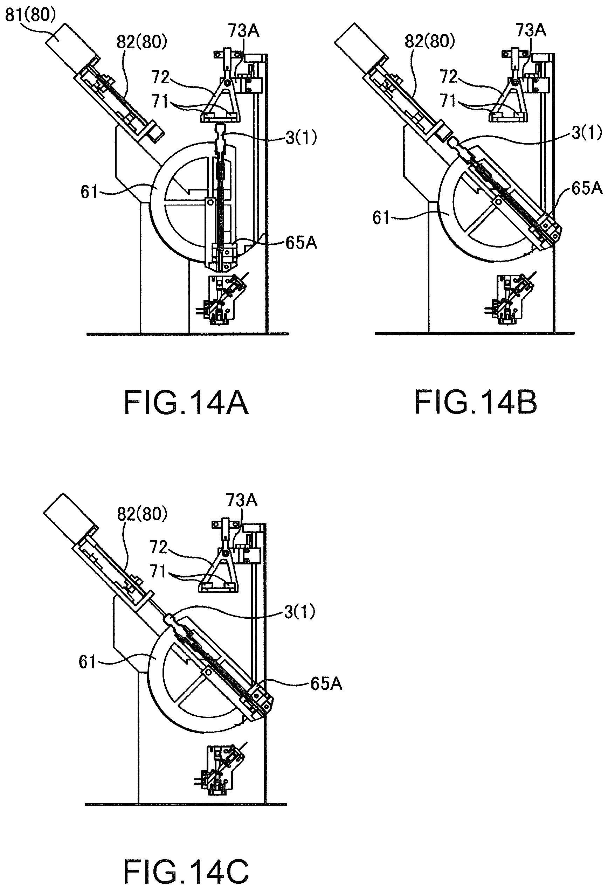

Figs. 14A to 14C are diagrams illustrating a droplet formation process. Fig. 14A is a diagram illustrating the state of the PCR device 50 when the magnets 71 are drawn up. Fig. 14B is a diagram illustrating the state where the rotary body 61 is rotated by 45 degrees. Fig. 14C is a diagram illustrating the state where the rod 82 of the pushing mechanism 80 pushes the plunger 10.

As shown in Fig. 14A, when the carriage 73A is at the retraction position, the elevating mechanism 73 does not come into contact with the cartridge 1 even when the cartridge 1 rotates. After this state is placed, the controller 90 rotates the rotary body 61 by 45 degrees.

As shown in Fig. 14B, when the rotary body 61 rotates by 45 degrees, the longitudinal direction of the cartridge 1 is in parallel with the movement direction of the rod 82 of the pushing mechanism 80. The controller 90 drives the motor for a plunger 81 to move the rod 82. When the rod 82 comes into contact with the mounting board 11A of the plunger 10 of the cartridge 1 and then moves further, the plunger 10 is pushed into the tube 20. The controller 90 moves the rod 82 until the state shown in Fig. 14C is placed and pushes the plunger 10 until the mounting board 11A of the plunger 10 comes into contact with the upper rim of the tube 20.

If the plunger 10 is pushed into the tube 20, the seal 12A of the rod-like portion 12 of the plunger 10 fits to the lower syringe 22 of the tube 20 (see Fig. 2B). Thereafter, if the plunger 10 is further pushed, the seal 12A slides inside the lower syringe 22. With this, the liquid (the third oil plug 48, the elution solution plug 47, and the like) on the downstream side of the tube 20 is pushed out to the flow path forming portion 35 of the PCR container 30 by an amount corresponding to the volume in which the seal 12A slides inside the lower syringe 22.

First, the third oil plug 48 of the tube 20 flows into the flow path forming portion 35. Since the flow path forming portion 35 is filled with oil, the inflow oil flows into the oil accommodating space 32A from the flow path forming portion 35, whereby the oil interface of the oil accommodating space 32A ascends. At this time, due to pressure loss of the lower seal portion 34B, the pressure of the liquid of the flow path forming portion 35 becomes higher than the outside pressure (the pressure of the oil accommodating space 32A). After the third oil plug 48 is pushed out from the tube 20, the elution solution plug 47 flows into the flow path forming portion 35 from the tube 20. Since the inner diameter of the flow path forming portion 35 is greater than the inner diameter of the capillary 23, the elution solution 47, which is made in the form of a plug in the tube 20, is formed into a droplet in the oil of the flow path forming portion 35.

The volume in which the seal 12A slides inside the lower syringe 22 (the amount of the liquid in the tube 20 that is pushed out from the downstream side) is greater than the total volume of the elution solution plug 47 and the third oil plug 48 in the tube 20. Therefore, after the elution solution plug 47 is pushed out from the tube 20, a portion of the second oil plug 46 is also pushed out to the flow path forming portion 35. With this, the elution solution 47 does not remain in the tube 20, and the entire elution solution plug 47 is formed into a droplet. Since a portion of the second oil plug 46 is pushed out from the downstream side of the tube 20, the elution solution 47 in the form of a droplet easily leaves the tube 20 (the elution solution 47 in the form of a droplet is not easily adsorbed onto the opening of the capillary 23).

The capillary 23 is designed such that the inner diameter of the terminal thereof (the diameter of the opening of the capillary 23) is comparatively small. Therefore, the elution solution 47, which has been formed in a droplet in the PCR container 30, is not easily adsorbed onto the opening of the capillary 23. The specific gravity of the elution solution 47 is greater than that of oil of the PCR container 30. For this reason, the elution solution 47 in the form of a droplet leaves the terminal of the capillary 23, passes through the flow path forming portion 35 as a flow path, and precipitates toward the bottom 35A. However, at this stage, since the flow path of the flow path forming portion 35 is inclined by 45 degrees, the elution solution 47 in the form of a droplet is easily adhered to the inner wall of the flow path forming portion 35. Therefore, the flow path of the flow path forming portion 35 should be returned to the vertical direction.

After the elution solution 47 in the form of a droplet is formed (after the plunger 10 is pushed), the controller 90 drives the motor for a plunger 81 in an opposite direction such that the rod 82 returns to the original position. In this state, even if the cartridge 1 rotates, the rod 82 of the pushing mechanism 80 does not come into contact with the cartridge 1. After this state is placed, the controller 90 returns the rotary body 61 to the reference position. When the rotary body 61 returns to the reference position, the flow path of the flow path forming portion 35 is in the vertical direction. Therefore, the elution solution 47 in the form of a droplet does not easily adhere to the inner wall of the flow path forming portion 35.

### 4. Thermal Cycle Process

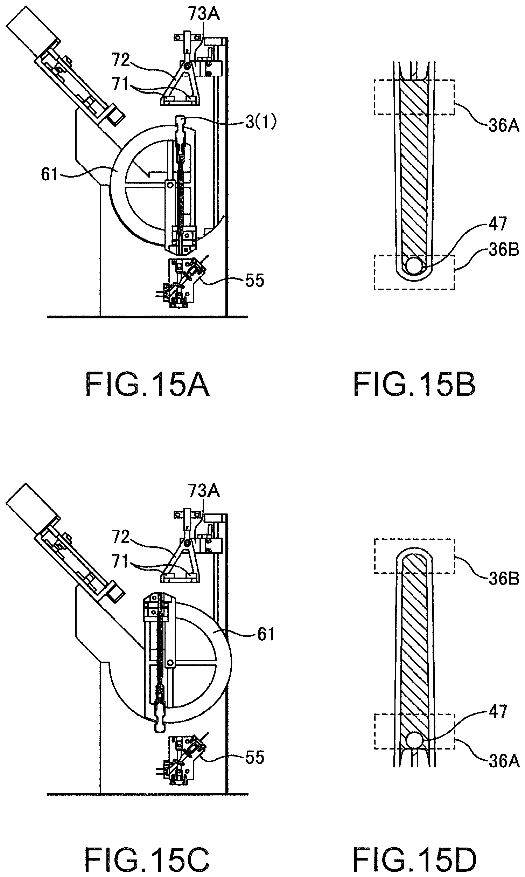

Figs. 15A to 15D are diagrams illustrating a thermal cycle process. Figs. 15A and 15B are diagrams illustrating a state where a temperature process on a low temperature side is performed on the elution solution 47. Figs. 15C and 15D are diagrams illustrating a state where a first temperature process is performed on the elution solution 47. Figs. 15A and 15C show the state of the PCR device 50, and Figs. 15B and 15D show the internal state of the flow path forming portion 35 of the PCR container 30.

If the cartridge 1 is fixed to the normal position in the mounting portion 62, the upstream side (high temperature region 36A) of the flow path forming portion 35 of the PCR container 30 faces the first heater 65A, and the downstream side (low temperature region 36B) of the flow path forming portion 35 of the PCR container 30 faces the second heater 65E. After the nucleic acid is eluted in the elution solution, the posture of the cartridge 1 is changed by the rotation of the rotary body 61, and before the elution solution 47 introduced into the PCR container 30 moves the opposite side to the side of the PCR container 30 to which the tube 20 is connected, for example, while reverse transcription is performed with RNA as a mold, the magnetic particles are retracted toward the first plug, or a droplet is formed, and the temperature of the first heater 65A is increased. During the thermal cycle process, the controller 90 maintains the liquid in the high temperature region 36A on the upstream side of the flow path forming portion 35 of the PCR container 30 at about 90°C to 100°C by the first heater 65A in the rotary body 61. The controller 90 heats the liquid in the low temperature region 36B on the downstream side of the flow path forming portion 35 to about 50°C to 75°C by the second heater 65E in the rotary body 61. With this, a temperature gradient is formed in the liquid in the flow path forming portion 35 of the PCR container 30 during the thermal cycle process. Since the mounting portion 62 and the heaters are provided in the rotary body 61, even if the rotary body 61 rotates, the positional relationship between the cartridge 1 and the heaters is maintained in this state.

During the thermal cycle process, the liquid in the PCR container 30 is heated. If air bubbles are formed in the liquid of the PCR container 30 due to heating of the liquid, the temperature of the liquid in the flow path forming portion 35 may become uneven, or movement (precipitation) of the elution solution 47 in the form of a droplet in the flow path forming portion 35 may be hindered. However, in this embodiment, due to pressure loss of the lower seal portion 34B, the pressure of the liquid of the flow path forming portion 35 is higher than the outside pressure. Therefore, air bubbles are not easily formed in the liquid of the PCR container 30.

As shown in Fig. 15A, when the rotary body 61 is at the reference position, as shown in Fig. 15B, the second heater 65E is positioned below the first heater 65A, and the bottom 35A of the PCR container 30 of the cartridge 1 becomes the lower side. Since the specific gravity of the elution solution 47 in the form of a droplet is greater than that of oil, the elution solution 47 in the form of a droplet precipitates inside the flow path forming portion 35. If precipitating inside the flow path forming portion 35, the elution solution 47 in the form of a droplet reaches the bottom 35A of the PCR container 30. The elution solution 47 stops to precipitate at the bottom 35A and stays in the low temperature region 36B. With this, the elution solution 47 in the form of a droplet moves to the low temperature region 36B. The controller 90 maintains the state shown in Fig. 15B for a predetermined amount of time and heats the elution solution 47 in the form of a droplet to about 50°C to 75°C in the low temperature region 36B (the elution solution 47 is subjected to a temperature process on the low temperature side). In the meantime, an extension reaction of the polymerase reaction occurs.

If the controller 90 drives the motor for rotation 66 to rotate the rotary body 61 by 180 degrees in the state of Figs. 15A and 15B, the cartridge 1 is moved upside down, and the first heater 65A and the second heater 65E are also moved upside down. That is, the first heater 65A is below the second heater 65E, and the bottom 35A of the PCR container 30 of the cartridge 1 becomes the upper side. After precipitating inside the flow path forming portion 35, the elution solution 47 in the form of a droplet reaches the terminal (the terminal of the capillary 23) of the tube 20. Then, the elution solution 47 stops to precipitate and stays in the high temperature region 36A. With this, the elution solution 47 in the form of a droplet moves to the high temperature region 36A. The controller 90 maintains the state of the state of Fig. 15D for a predetermined amount of time and heats the elution solution 47 in the form of a droplet to about 90°C to 100°C in the high temperature region 36A (a temperature process on the high temperature side is performed). In the meantime, a denaturation reaction of the polymerase reaction occurs.

If the controller 90 drives the motor for rotation 66 to rotate the rotary body 61 by -180 degrees in the state of Fig. 15C, the state of Fig. 15A is returned. In this state, if the elution solution 47 in the form of a droplet precipitates inside the flow path forming portion 35, the elution solution 47 in the form of a droplet moves to the low temperature region 36B and is heated again to about 50°C to 75°C in the low temperature region 36B (a temperature process on the low temperature side is performed). Since the capillary 23 is designed such that the inner diameter of the terminal thereof (the diameter of the opening of the capillary 23) is comparatively small, the elution solution 47 is not easily adsorbed onto the opening of the capillary 23. Therefore, the rotary body 61 rotates by -180 degrees in the state of Fig. 15B, the elution solution 47 in the form of a droplet leaves the tube 20 and precipitates toward the bottom 35A of the PCR container 30 without being adsorbed onto the opening of the capillary 23.

The controller 90 drives the motor for rotation 66 such that the rotation position of the rotary body 61 is placed in the state of Fig. 15A and the state of Fig. 15C. The controller 90 repeats this operation for a predetermined number of cycles. With this, the PCR device 50 can perform the thermal cycle process of PCR on the elution solution 47.

The storage device of the controller 90 stores thermal cycle information of the temperature of the first heater 65A, the temperature of the second heater 65E, the time during which the state of Fig. 15A is held, the time during which the state of Fig. 15C is held, and the number of cycles (the number of repetitions of the state of Fig. 15A and the state of Fig. 15C). The controller 90 executes the above process according to the thermal cycle information.

### 5. Fluorescence Measurement

As shown in Fig. 15A, when the rotary body 61 is at the reference position, the fluorescence measuring instrument 55 faces the bottom 35A of the PCR container 30 of the cartridge 1. For this reason, when measuring fluorescence of the elution solution 47, the controller 90 causes the fluorescence measuring instrument 55 to measure fluorescence intensity of the elution solution 47 at the bottom 35A of the PCR container 3 0 from the lower opening of the insertion hole 64A of the second heater 65E in a state where the rotary body 61 is at the reference position.

Immediately after the rotary body 61 rotates by 180 degrees and is at the reference position, the elution solution 47 in the form of a droplet precipitates inside the flow path forming portion 35 of the PCR container 30, and the elution solution 47 in the form of a droplet may not reach the bottom 35A of the PCR container 30. For this reason, it is desirable that the controller 90 measures fluorescence intensity when a predetermined time elapses after the rotation position of the rotary body 61 is placed in the state of Fig. 15A (immediately before the rotary body 61 is rotated from the state of Fig. 15A). Alternatively, the controller 90 may cause the fluorescence measuring instrument 55 to measure fluorescence intensity for a predetermined time after the rotary body 61 is at the reference position and may store a time history of fluorescence intensity.

### Overview

The PCR device 50 is provided with the first heater 65A as a heater which heats the elution solution plug 47. If the elution solution plug 47 of the cartridge 1 is heated, for example, to about 65°C by the first heater 65A, the liberation of the nucleic acid from the magnetic particles can be accelerated. The first heater 65A functions as a first heater which heats the upstream side (high temperature region 36A) of the flow path forming portion 35 of the PCR container 30, not a dedicated heater which heats the elution solution plug 47. Here, the elution solution plug 47 and the upstream side (high temperature region 36A) of the flow path forming portion 35 of the PCR container 30 are positioned adjacent to each other through the plug 48. For this reason, when a heater which heats the elution solution plug 47 and a heater which heats the upstream side (high temperature region 36A) of the flow path forming portion 35 of the PCR container 30 are separately provided, these heaters should be disposed close together, and heat transmitted from one heater may increase the temperature of the other heater to be equal to or higher than a set temperature. When such heat interference occurs, it is difficult to appropriately control the temperature of the elution solution plug 47 of the cartridge 1 and the temperature of the upstream side (high temperature region 36A) of the flow path forming portion 35 of the PCR container 30. In contrast, the PCR device 50 according to this embodiment uses a single (first heater 65A) to heat the elution solution plug 47 and the upstream side (high temperature region 36A) of the flow path forming portion 35 of the PCR container 30, whereby the temperature of the heater can be maintained stably.

If two heaters are integrated, the entire heater can be reduced in weight. Therefore, it is possible to reduce the load of the motor for rotation 66. During the thermal cycle process, the temperature of the first heater 65A should be set to a higher temperature than when the nucleic acid is liberated into the elution solution plug 47. However, the temperature may be increased until the thermal cycle process starts after the nucleic acid is eluted.

### Second Embodiment

In a second embodiment, a cartridge 1 having a configuration different from that in the first embodiment is mounted on a PCR device 50.

### Cartridge 1

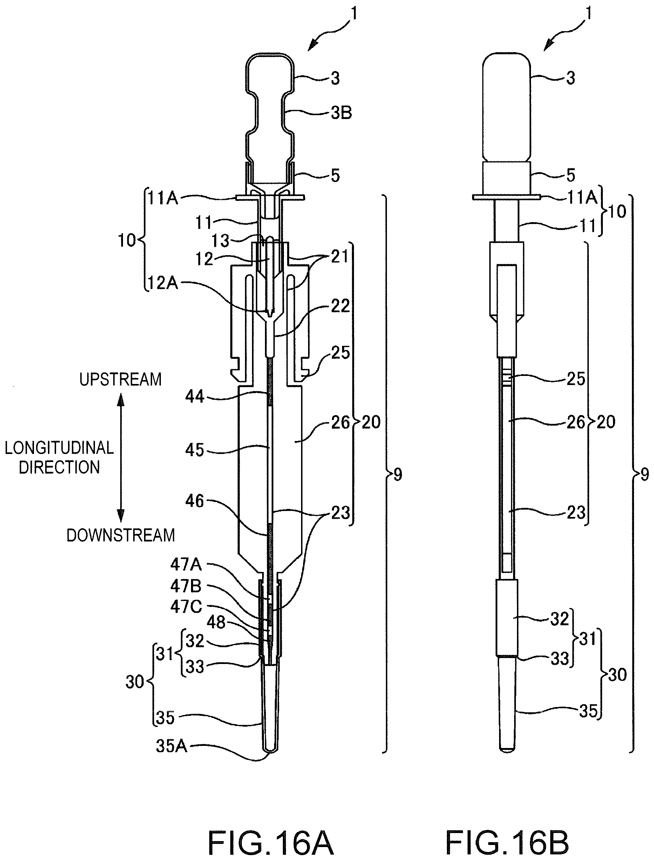

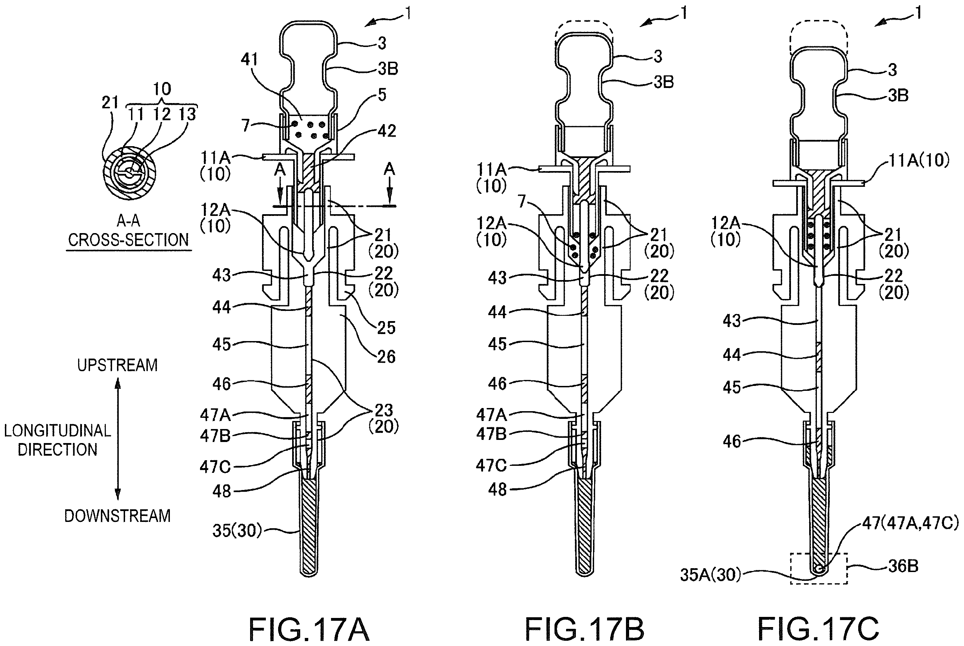

Figs. 16A and 16B are diagrams illustrating the cartridge 1 of the second embodiment. Fig. 17A is a diagram illustrating the initial state of the cartridge 1 of the second embodiment. Fig. 17B is a side view showing a state where a plunger 10 is pushed in the state of Fig. 17A such that the seal 12A comes into contact with the lower syringe 22. Fig. 17C is a diagram illustrating the cartridge 1 of the second embodiment after the plunger 10 is pushed. A tube 20 of the cartridge 1 of the second embodiment includes an elution solution plug 47A, an oil plug 47B, and a nucleic acid amplification reaction solution plug 47C, instead of the elution solution plug 47 of the first embodiment. The oil plug 47B prevents the elution solution plug 47A and the nucleic acid amplification reaction solution plug 47C as water-soluble plugs on both sides from being mixed with each other.

The cartridge 1 is a container in which a nucleic acid elution process for causing nucleic acid to be eluted from magnetic particles 7 bound to the nucleic acid is performed. The cartridge 1 is also a container in which a thermal cycle process for a polymerase reaction is performed on a PCR solution as a mixed solution of the elution solution 47A and the nucleic acid amplification reaction solution 47C.

The shapes of a tank 3 of the cartridge 1 and a cartridge main body 9 are the same as those in the first embodiment. A dissolution solution 41 of the tank 3 of the cartridge 1 is the same as that in the first embodiment. The oil 42, the first washing solution 43, the first oil plug 44, the washing solution plug 45 (second washing solution), and the second oil plug 46 of the cartridge main body 9 are the same as those in the first embodiment. For this reason, description thereof will be omitted.

The lower end portion of the capillary 23 is inserted into the PCR container 30. With this, if the nucleic acid amplification reaction solution plug 47C and the elution solution plug 47A in the tube 20 are pushed out from the tube 20, the nucleic acid amplification reaction solution 47C and the elution solution 47A can be introduced into the PCR container 30.

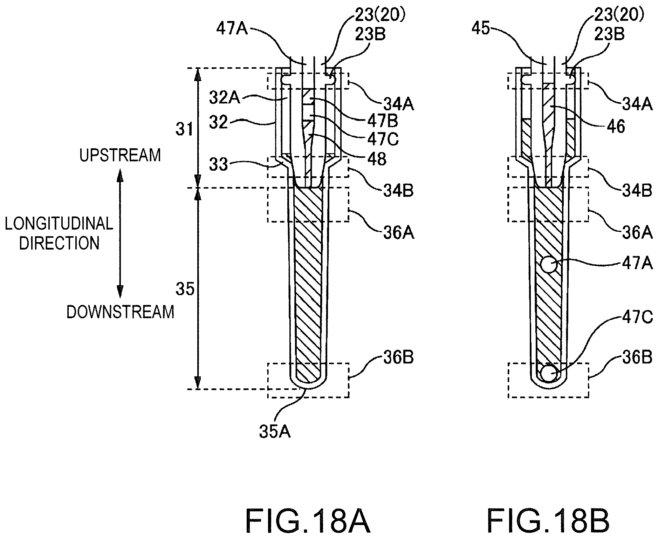

Figs. 18A and 18B are diagrams illustrating the periphery of the PCR container 30 of the second embodiment. Fig. 18A is a diagram illustrating an initial state. Fig. 18B is a diagram showing a state after the plunger 10 is pushed. Hereinafter, description will be provided also referring to Figs. 17A to 17C.

The PCR container 30 is a container which receives a liquid pushed out from the tube 20. The PCR container 30 is also a container which accommodates a PCR solution as a mixed solution of the nucleic acid amplification reaction solution 47C and the elution solution 47A during a thermal cycle process.

If the plunger 10 is pushed, first, the third oil plug 48 of the tube 20 flows into the flow path forming portion 35, and the inflow oil then flows into an oil accommodating space 32A from the flow path forming portion 35, whereby the oil interface of the oil accommodating space 32A ascends. At this time, due to pressure loss of a lower seal portion 34B, the pressure of a liquid of the flow path forming portion 35 increases.

After the third oil plug 48 is pushed out from the tube 20, the nucleic acid amplification reaction solution plug 47C flows into the flow path forming portion 35 from the tube. Since the inner diameter of the flow path forming portion 35 is greater than the inner diameter of the capillary 23, the nucleic acid amplification reaction solution 47C, which has been in the form of a plug (a column) in the tube 20, becomes a droplet in the flow path forming portion 35. Since the nucleic acid amplification reaction solution 47C has a specific gravity greater than oil, the nucleic acid amplification reaction solution 47C in the form of a droplet precipitates.

After the nucleic acid amplification reaction solution plug 47C is pushed out from the tube 20, the oil plug 47B flows into the flow path forming portion 35. After the oil plug 47B is pushed out from the tube 20, the elution solution plug 47A flows into the flow path forming portion 35 from the tube 20. Since the inner diameter of the flow path forming portion 35 is greater than the inner diameter of the capillary 23, the elution solution 47A, which has been in the form of a plug (a column) in the tube 20, becomes a droplet in the flow path forming portion 35. Since the elution solution 47A has a specific gravity greater than oil, the elution solution 47A in the form of a droplet precipitates. If the elution solution 47A in the form of a droplet precipitates, the elution solution 47A is merged with the nucleic acid amplification reaction solution 47C in the form of a droplet, which already precipitates at the bottom of the flow path forming portion 35, in oil and becomes a PCR solution 47 in the form of a droplet.

### Description of Operation of PCR Device 50

The structure of the PCR device 50 of the second embodiment is the same as that in the first embodiment. For this reason, description of the structure of the PCR device 50 (rotating mechanism 60, magnet moving mechanism 70, pushing mechanism 80, fluorescence measuring instrument 55, controller 90, and the like) will be omitted. Here, the operation of the PCR device 50 when the cartridge 1 of the second embodiment is mounted will be described.

### 1. Operation for Mounting Cartridge 1

The state where the cartridge 1 of the second embodiment is mounted is the same as the state of Figs. 9A to 9D of the first embodiment. In the second embodiment, as shown in Fig. 9C, if the cartridge 1 is fixed to the normal position in the mounting portion 62, the elution solution plug 47A of the tube 20 faces the first heater 65A.

### 2. Nucleic Acid Elution Process

The state where a nucleic acid elution process of the second embodiment is performed is the same as the state of Figs. 11A to 11C of the first embodiment.

In the second embodiment, as shown in Fig. 11B, after the magnets 71 move to the position (the height of the elution solution plug 47A) facing the elution solution plug 47A, the controller 90 stops the motor for elevation 73B to stop the movement of the magnets 71 in the up-down direction and performs the process for 30 seconds at 50°C. Therefore, the nucleic acid bonded to the magnetic particles 7 is liberated into the solution of the elution solution plug 47, and a reverse transcription reaction proceeds. If the elution solution 47 is heated, the elution of nucleic acid from the magnetic particles 7 and the reverse transcription are accelerated.

After the nucleic acid is eluted in the elution solution plug 47A, the controller 90 drives the motor for elevation 73B in an opposite direction such that the carriage 73 slowly moves upward to slowly move the magnets 71 upward. If the magnets 71 move upward in the state shown in Fig. 11B, the magnetic particles 7 move from the elution solution plug 47A to the second oil plug 46, whereby the magnetic particles 7 are removed from the elution solution plug 47A.

In the second embodiment, the magnetic particles 7 do not come into contact with the nucleic acid amplification reaction solution 47C. For this reason, the enzyme of the nucleic acid amplification reaction solution 47C is prevented from being adsorbed onto the magnetic particles 7, and the reaction is prevented from being hindered. Therefore, in the second embodiment, it is possible to perform the nucleic acid elution process under high efficiency conditions (high temperature, high-frequency oscillation, increase in weight of magnetic particles, and the like) compared to the first embodiment.

### 3. Droplet Forming Process

A state where a droplet forming process of the second embodiment is performed is the same as the state of Figs. 14A to 14C of the first embodiment.

If the plunger 10 is pushed, first, the third oil plug 48 of the tube 2 0 flows into the flow path forming portion 35, and the inflow oil flows into the oil accommodating space 32A from the flow path forming portion 35, whereby the oil interface of the oil accommodating space 32A ascends. At this time, due to pressure loss of the lower seal portion 34B, the pressure of a liquid of the flow path forming portion 35 increases.

After the third oil plug 48 is pushed out from the tube 20, the nucleic acid amplification reaction solution plug 47C flows into the flow path forming portion 35 from the tube. Since the inner diameter of the flow path forming portion 35 is greater than the inner diameter of the capillary 23, the nucleic acid amplification reaction solution 47C, which has been in the form of a plug (a column) in the tube 20, becomes a droplet in the flow path forming portion 35. Since the nucleic acid amplification reaction solution 47C has a specific gravity greater than oil, the nucleic acid amplification reaction solution 47C in the form of a droplet precipitates.

After the nucleic acid amplification reaction solution plug 47C is pushed out from the tube 20, the oil plug 47B flows into the flow path forming portion 35. After the oil plug 47B is pushed out from the tube 20, the elution solution plug 47A flows into the flow path forming portion 35 from the tube 20. Since the inner diameter of the flow path forming portion 35 is greater than the inner diameter of the capillary 23, the elution solution 47A, which has been in the form of a plug (a column) in the tube 20, becomes a droplet in the flow path forming portion 35. Since the elution solution 47A has a specific gravity greater than oil, the elution solution 47A in the form of a droplet precipitates. If the elution solution 47A in the form of a droplet precipitates, the elution solution 47A is merged with the nucleic acid amplification reaction solution 47C in the form of a droplet, which already precipitates at the bottom of the flow path forming portion 35, in oil and becomes a PCR solution 47 in the form of a droplet.

The elution solution 47A and the nucleic acid amplification reaction solution 47C in the form of droplets are small. Therefore, two droplets may not be merged at the bottom of the flow path forming portion 35, and may be separated from each other. Accordingly, after the elution solution 47A in the form of a droplet precipitates at the bottom of the flow path forming portion 35, the controller 90 drives the rotating mechanism 60 little by little to vibrate the rotary body 61. With this, even if two droplets are separated from each other at the bottom of the flow path forming portion 35, the two droplets are merged by the vibration of the cartridge 1 and become the PCR solution 47 in the form of a droplet. If droplets are heated by the first heater 65A or the second heater 65E to reduce viscosity of the droplets, two droplets may be easily merged.

After the droplet forming process, a thermal cycle process or fluorescence measurement is performed. The thermal cycle process and fluorescence measurement of the second embodiment are the same as those in the first embodiment, thus, description thereof will be omitted.

In the second embodiment, the PCR device 50 includes the rotary body 61 which has a mounting portion (fixing portion 63 and insertion hole 64A), on which the cartridge 1 is mounted, and heaters for PCR (first heater 65A and second heater 65E) which form a temperature gradient inside the PCR container 30 as a nucleic acid amplification reaction container. The posture of the cartridge 1 is changed by the rotation of the rotary body 61 and the elution solution 47 in the form of a droplet introduced from the tube 20 moves inside the PCR container 30. With this, the posture of the PCR container 30 is changed along with the tube 20 in which the nucleic acid elution process is performed, whereby the polymerase reaction can be performed. Therefore, the processing time can be shortened.

### Other Aspects

The above embodiments are for ease of understanding of the invention, and the invention is not limited to these embodiments. Needless to say, the invention can be modified or improved without departing from the spirit of the invention, and the invention also includes the equivalents thereof.

### Regarding PCR Device

The PCR device 50 changes the posture of the cartridge 1 by a predetermined number of cycles to perform a thermal cycle process and moves the PCR container 30 upside down. However, the number of changes of the posture of the cartridge 1 is not limited to multiple times and may be once.

In the above-described PCR device, the rotary shaft of the rotary body is positioned closer to the tube than to the PCR container. However, the position of the rotary shaft of the rotary body is not limited thereto as long as the droplet can move inside the PCR container when the posture of the cartridge is changed due to the rotation of the rotary body.

In the above-described PCR device, the fixing portion 63, in which a notch is formed, and the insertion hole 64A constitutes the mounting portion of the cartridge. However, the mounting portion is not limited to this configuration as long as the cartridge can be mounted on the rotary body. The mounting portion may have a structure in which the cartridge is fixed to the rotary body by only fixing the tube side, or may have a structure in which the cartridge is fixed to the rotary body by only fixing the PCR container side. However, the mounting portion should have a structure in which the cartridge is stably fixed to the rotary body even if the posture of the cartridge is changed due to the rotation of the rotary body.

The above-described PCR device 50 has the first heater 65A and the second heater 65E as the heaters for PCR. However, the device is not limited to this configuration as long as a temperature gradient can be formed inside the PCR container as a container for a nucleic acid amplification reaction. For example, the heater may be provided only on a high temperature side. Alternatively, a heater may be provided on a high temperature side, and a cooler may be provided on a low temperature side.

In the above-described PCR device 50, although the heaters for PCR (first heater 65A and second heater 65E) are provided in the rotary body, the heaters for PCR may be provided outside the rotary body as long as a temperature gradient can be formed inside the PCR container as a nucleic acid amplification reaction container. For example, the PCR device may be provided with a first heater for PCR which faces the PCR container when the rotary body 61 is at the reference position as shown in Fig. 15A and a second heater for PCR which faces the PCR container when the rotary body is rotated by 180 degrees as shown in Fig. 15C. Even in this structure, a temperature gradient can be formed inside the PCR container as a nucleic acid amplification reaction container. However, it is desirable that the heaters for PCR are provided in the rotary body since the positional relationship between the PCR container of the cartridge and the heaters can be maintained regardless of the rotation position of the rotary body.

The PCR device 50 may not have the magnet moving mechanism which moves the magnets along the tube. In this case, for example, the operator may grab and move the magnets along the tube. However, it is desirable that the PCR device 50 has the magnet moving mechanism since the movement speed and the like of the magnetic particles as nucleic acid-binding solid-phase carriers may be changed by the operator.

The magnet moving mechanism 70 of the PCR device 50 may not have the elevating mechanism 75. In this case, although the magnets cannot oscillate, the magnets can be moved along the tube. Therefore, the magnetic particles bonded to nucleic acid can be moved to the plug containing the elution solution.

The PCR device 50 may not have the pushing mechanism 80. In this case, for example, the operator may push the plunger of the cartridge by hand. When the plunger is not provided in the cartridge, the operator may deform the tank, whereby the inside of the tank may be pressed to push out the liquid from the tube to the PCR container.

The PCR device 50 may not have the fluorescence measuring instrument. In this case, the device cannot perform real time PCR but can perform a polymerase reaction for amplifying nucleic acid.

### Regarding Elution Solution Plug 47A

The elution solution plug 47A may be divided into a reverse transcription reaction solution plug and an elution solution plug. In this case, nucleic acid-binding solid-phase carriers washed with a washing solution are moved to the reverse transcription reaction solution plug and are subjected to a reverse transcription reaction in the reverse transcription solution plug. This reaction can be performed under conditions suitable for a reverse transcription enzyme to be used. For example, if the reverse transcription reaction solution is heated to 30°C to 50°C and preferably 42°C to 45°C, and the nucleic acid-binding solid-phase carriers are held in the reverse transcription reaction solution for a given time, the reverse transcription reaction can occur in a state where RNA is bound to the carriers. A heating method is not particularly limited, and examples of the heating method include a method of bringing a heat medium, such as a heat block, into contact with the position of the tube corresponding to the reverse transcription transcription solution plug, a method of using a heat source, such as a heater, a method using electromagnetic heating, and the like. Alternatively, the first heater for heating the elution solution plug may be extended to the position corresponding to the reverse transcription transcription solution plug, and heating may be performed by the first heater. The holding time can also be appropriately selected by the operator. For example, the carriers may be held for 10 seconds to 5 minutes, and preferably 30 seconds to 1 minute. cDNA synthesized at this stage is bound to the solid-phase carriers in a state of being bound to RNA.

Thereafter, the nucleic acid-binding solid-phase carriers are moved to the elution solution plug. Here, in order to cause the nucleic acid, particularly cDNA, to be efficiently liberated from the nucleic acid-binding solid-phase carriers, it is preferable to heat the elution solution plug. The heating method is not particularly limited, and the same methods as those used for heating the reverse transcription reaction solution plug can be used. The heating temperature may be higher than 40°C, and is preferably equal to or higher than 50°C and more preferably equal to or higher than 60°C. The upper limit of the heating temperature is not particularly limited, but is preferably equal to or lower than 75°C, more preferably equal to or lower than 70°C, and most preferably equal to or lower than 65°C.

After the nucleic acid is liberated from the nucleic acid-binding solid-phase carriers, the nucleic acid-binding solid-phase carriers are moved upward from the elution solution plug using the magnets. The nucleic acid-binding solid-phase carriers may be moved to any position as long as the nucleic acid-binding solid-phase carriers are not mixed into the elution solution plug.

If the elution solution plug is divided into the reverse transcription reaction solution plug and the elution solution plug in this way, the reverse transcription reaction and the nucleic acid elution can be performed under high-efficiency conditions.

## Claims

1. A nucleic acid amplification reaction device comprising:
a rotary body (61) on which a cartridge (1) is mountable, the cartridge (1) including
- a tube (20) having, in this order in the inside thereof, a first plug (44) formed of oil, a fourth plug (47) formed of an elution solution which causes the nucleic acid to be eluted from magnetic particles (7) bound to the nucleic acid, and a fifth plug (48) formed of oil, and
- a nucleic acid amplification reaction container (30) which communicates with the side of the tube (20) on which the fifth plug (48) is disposed and in which a nucleic acid amplification reaction reagent is held in oil; and
a single first heater (65A) which is able to heat the end portion of the tube (20) on the side communicating with the nucleic acid amplification reaction container (30) and to heat a first region as the end portion of the nucleic acid amplification reaction container (30) on the side communicating with the tube (20),
wherein the nucleic acid amplification reaction device is configured such that the posture of the cartridge (1) is changed by the rotation of the rotary body (61) and droplets containing the elution solution introduced from the tube (20) move in the inside of the nucleic acid amplification reaction container (30).

2. The nucleic acid amplification reaction device of claim 1, wherein the tube (20) included in the cartridge (1) has, in this order in the inside thereof, the first plug (44) formed of oil, a second plug (45) formed of a first washing solution which undergoes phase separation from oil and washes magnetic particles (7) bound to nucleic acid, a third plug (46) formed of oil, the fourth plug (47), formed of an elution solution which undergoes phase separation from oil and causes the nucleic acid to be eluted from the magnetic particles (7) bound to the nucleic acid, and the fifth plug (48) formed of oil.

3. The nucleic acid amplification reaction device according to claim 1 or 2, further comprising a second heater (65E) which is able to heat a second region as the other end portion of the nucleic acid amplification reaction container (30).

4. The nucleic acid amplification reaction device according to any one of the preceding claims, further comprising a control unit (90) configured to control the temperature of the first heater (65A) such that the temperature of the first heater (65A) is set to a first temperature during the elution of the nucleic acid and the temperature of the first heater (65A) becomes a second temperature higher than the first temperature after the elution of the nucleic acid before the posture of the cartridge (1) is changed by the rotation of the rotary body (61) and droplets containing the elution solution introduced from the tube (20) into the nucleic acid amplification reaction container (30) move to the second region.

5. The nucleic acid amplification reaction device according to any one of the preceding claims, further comprising a pushing mechanism (80) which is attached to an opening of the tube (20) on a side different from the side communicating with the nucleic acid amplification reaction container (30) and configured to push out liquid from the side of the tube (20) communicating with the nucleic acid amplification reaction container (30) to the nucleic acid amplification reaction container (30).

6. A method of amplifying nucleic acid using the nucleic acid amplification reaction device according to any one of the preceding claims, the method comprising:
causing the nucleic acid to be eluted from magnetic particles (7) bound to the nucleic acid in a fourth plug (47) formed of an elution solution;
retracting the magnetic particles (7) from the fourth plug (47) toward the first plug (44) after the elution of the nucleic acid; and
performing a droplet forming process to push out the elution solution from the tube (20) to the nucleic acid amplification reaction container (30) after the retraction of the magnetic particles (7),
wherein the temperature of the first heater (65A) is set to a first temperature during the elution of the nucleic acid, and
the temperature of the first heater (65A) is changed to a second temperature such that the temperature of the first heater (65A) becomes the second temperature after the elution of the nucleic acid before the posture of the cartridge (1) is changed by the rotation of the rotary body (61) and droplets containing the elution solution inside the nucleic acid amplification reaction container (30) move from a first region to a second region.

7. The method according to claim 6, wherein, while the retraction of the magnetic particles (7) or the droplet forming process is performed, the temperature of the first heater (65A) is increased from the first temperature to the second temperature.

8. The method according to claim 6 or 7,
wherein the nucleic acid is RNA, and the elution solution contains a reverse transcription reaction reagent, and
the method further comprises:
performing reverse transcription to cause the RNA to be reversely transcribed in the elution solution.

9. The method according to any one of claims 6 to 8, wherein, while the retraction of the magnetic particles (7), the droplet forming process, or the reverse transcription is performed, the temperature of the first heater (65A) is increased from the first temperature to the second temperature.

## Patentansprüche

1. Vorrichtung für eine Nukleinsäureamplifikationsreaktion, umfassend:
einen Rotationskörper (61), an welchem eine Patrone (1) anbringbar ist, wobei die Patrone (1) beinhaltet
- einen Schlauch (20) der in dieser Reihenfolge in seinem Inneren aufweist einen ersten Stopfen (44) der aus einem Öl ausgebildet ist, einen vierten Stopfen (47) der aus einer Waschlösung ausgebildet ist, die verursacht, dass die Nukleinsäure von magnetischen Partikeln (7), die an die Nukleinsäure gebunden sind, gereinigt wird, und einen fünften Stopfen (48), der aus Öl ausgebildet ist, und
- einen Behälter (30) für eine Nukleinsäureamplifikationsreaktion, der mit der Seite des Schlauchs (20) verbunden ist, an welcher der fünfte Stopfen (48) angeordnet ist, und in welcher ein Reagenz für eine Nukleinsäureamplifikationsreaktion in Öl gehalten ist; und
eine einzelne erste Heizung (65A), die dazu in der Lage ist, einen Endabschnitt des Schlauchs (20) an der Seite des Behälters (30) für eine Nukleinsäureamplifikationsreaktion zu heizen und einen ersten Bereich als den Endabschnitt des Behälters (30) für eine Nukleinsäureamplifikationsreaktion an der Seite zu heizen, die mit dem Schlauch (20) verbunden ist,
wobei die Vorrichtung für die Nukleinsäureamplifikationsreaktion so ausgestaltet ist, dass eine Lage der Patrone (1) durch die Rotation des Rotationskörpers (61) geändert wird und Tropfen, welche die Waschlösung beinhalten, die von dem Schlauch (20) eingeführt werden, sich zu dem inneren des Behälters (30) für eine Nukleinsäureamplifikationsreaktion bewegen.

2. Vorrichtung für eine Nukleinsäureamplifikationsreaktion nach Anspruch 1, wobei der Schlauch (20), der in der Patrone (1) enthalten ist, in dieser Reihenfolge in seinem Inneren aufweist den ersten Stopfen (44), der aus Öl ausgebildet ist, einen zweiten Stopfen (45), der aus einer ersten Waschlösung ausgebildet ist, die eine Phasentrennung von Öl durchläuft und magnetische Partikel (7), die an die Nukleinsäure angebunden sind, auswäscht, einen dritten Stopfen (46), der aus Öl ausgebildet ist, den vierten Stopfen (47), der aus einer Waschlösung ausgebildet ist, welche eine Phasentrennung von Öl durchläuft und verursacht, dass die Nukleinsäuren von den magnetischen Partikeln (7), die an die Nukleinsäure angebunden sind, gereinigt wird, und den fünften Stopfen (48), der aus Öl ausgebildet ist.

3. Vorrichtung für eine Nukleinsäureamplifikationsreaktion nach Anspruch 1 oder 2, ferner eine zweite Heizung (65B) umfassend, die dazu in der Lage ist, einen zweiten Bereich als den anderen Endabschnitt des Behälters (30) für eine Nukleinsäureamplifikationsreaktion zu heizen.

4. Vorrichtung für eine Nukleinsäureamplifikationsreaktion nach einem der vorhergehenden Ansprüche, ferner umfassend eine Steuerungseinheit (90), die dazu ausgestaltet ist, die Temperatur der ersten Heizung (65A) zu steuern, sodass die Temperatur der ersten Heizung (65A) auf eine erste Temperatur während des Waschens der Nukleinsäure gesetzt ist und die Temperatur der ersten Heizung (65A) eine zweite Temperatur wird, die höher als die erste Temperatur ist, nach dem Waschen der Nukleinsäure, bevor die Lage der Patrone (1) durch die Rotation des Rotationskörpers (61) geändert wird und Tropfen, welche die Waschlösung enthalten, sich von dem Schlauch (20) in den Behälter (30) für die Nukleinsäureamplifikationsreaktion zu einem zweiten Bereich bewegen.

5. Vorrichtung für eine Nukleinsäureamplifikationsreaktion, nach einem der vorhergehenden Ansprüche, ferner einen Drückmechanismus (80) umfassend, der an einer Öffnung des Schlauchs (20) an einer Seite angebracht ist, die sich von der Seite unterscheidet, die mit dem Behälter (30) für die Nukleinsäureamplifikationsreaktion verbunden ist, und dazu ausgestaltet ist, eine Flüssigkeit von der Seite des Schlauchs (20), die mit dem Behälter (30) für die Nukleinsäureamplifikationsreaktion verbunden ist, zu dem Behälter (30) für die Nukleinsäureamplifikationsreaktion auszudrücken.

6. Verfahren für eine Nukleinsäureamplifikation unter Verwendung der Vorrichtung für eine Nukleinsäureamplifikationsreaktion nach einem der vorhergehenden Ansprüche, wobei das Verfahren umfasst:
Verursachen, dass die Nukleinsäure von den magnetischen Partikeln (7), die an die Nukleinsäure angebunden sind, in einem vierten Stopfen (47), der aus einer Waschlösung ausgebildet ist, gereinigt werden;
Zurückziehen der magnetischen Partikel (7) von dem vierten Stopfen (47) zu dem ersten Stoffen (44) nach dem Waschen der Nukleinsäure; und
Ausführen eines Ausbildungsprozesses für einen Tropfen, um die Waschlösung von dem Schlauch (20) zu dem Behälter (30) für eine Nukleinsäureamplifikationsreaktion nach dem Zurückziehen der magnetischen Partikel (7) auszudrücken,
wobei die Temperatur der ersten Heizung (65A) auf eine erste Temperatur während der Reinigung der Nukleinsäure gesetzt ist, und
die Temperatur der ersten Heizung (65A) zu der zweiten Temperatur geändert wird, so dass die Temperatur der ersten Heizung (65A) die zweite Temperatur nach dem Waschen der Nukleinsäure wird, bevor die Lage der Patrone (1) durch die Rotation des Rotationskörpers (61) geändert wird und Tropfen, welche die Waschlösung enthalten, sich von dem Schlauch (20) in den Behälter (30) für die Nukleinsäureamplifikationsreaktion zu einem zweiten Bereich bewegen.

7. Verfahren nach Anspruch 6, wobei während dem Zurückziehen der magnetischen Partikel (7) oder der Ausbildungsprozess für den Tropfen durchgeführt wird, die Temperatur der ersten Heizung (65A) von der ersten Temperatur zu der zweiten Temperatur erhöht wird.

8. Verfahren nach Anspruch 6 oder 7,
wobei die Nukleinsäure RNA ist und die Waschlösung eine Reagenz für eine umgekehrte Transkriptionsreaktion enthält, und
das Verfahren ferner umfasst:
Durchführen der umgekehrten Transkriptionsreaktion, um zu verursachen, dass die RNA umgekehrt in der Waschlösung transkribiert ist.

9. Verfahren nach einem der Ansprüche 6-8, wobei, während dem Zurückziehen der magnetischen Partikel (7), des Ausbildungsprozesses für den Tropfen oder die umgekehrte Transkription durchgeführt wird, die Temperatur der ersten Heizung (65A) von der ersten Temperatur zu der zweiten Temperatur erhöht wird.

## Revendications

1. Dispositif de réaction d'amplification d'acide nucléique comprenant :
un corps rotatif (61) sur lequel une cartouche (1) peut être montée, la cartouche (1) incluant
- un tube (20) ayant, dans cet ordre à l'intérieur de celui-ci, un premier bouchon (44) formé d'huile, un quatrième bouchon (47) formé d'une solution d'élution qui conduit l'acide nucléique à être élué à partir de particules magnétiques (7) liées à l'acide nucléique, et un cinquième bouchon (48) formé d'huile, et
- un conteneur de réaction d'amplification d'acide nucléique (30) qui communique avec le côté du tube (20) sur lequel le cinquième bouchon (48) est disposé et dans lequel un réactif de réaction d'amplification d'acide nucléique est tenu dans de l'huile; et
un seul premier appareil de chauffage (65A) qui est capable de chauffer la portion terminale du tube (20) sur le côté communicant avec le conteneur de réaction d'amplification d'acide nucléique (30) et de chauffer une première région comme la portion terminale du conteneur de réaction d'amplification d'acide nucléique (30) sur le côté communicant avec le tube (20),
dans lequel le dispositif de réaction d'amplification d'acide nucléique est configuré de sorte que la posture de la cartouche (1) est changée par la rotation du corps rotatif (61) et des gouttelettes contenant la solution d'élution introduite depuis le tube (20) se déplacent à l'intérieur du conteneur de réaction d'amplification d'acide nucléique (30).

2. Dispositif de réaction d'amplification d'acide nucléique selon la revendication 1, dans lequel le tube (20) inclut dans la cartouche (1) a, dans cet ordre à l'intérieur de celui-ci, le premier bouchon (44) formé d'huile, un deuxième bouchon (45) formé d'une première solution de nettoyage qui subit une séparation de phase de l'huile et lave des particules magnétiques (7) liées à l'acide nucléique, un troisième bouchon (46) formé d'huile, le quatrième bouchon (47), formé d'une solution d'élution qui subit une séparation de phase de l'huile et conduit l'acide nucléique à être élué à partir des particules magnétiques (7) liées à l'acide nucléique, et le cinquième bouchon (48) formé d'huile.

3. Dispositif de réaction d'amplification d'acide nucléique selon la revendication 1 ou 2, comprenant en outre un deuxième appareil de chauffage (65E) qui est capable de chauffer une deuxième région comme l'autre portion terminale du conteneur de réaction d'amplification d'acide nucléique (30).

4. Dispositif de réaction d'amplification d'acide nucléique selon l'une quelconque des revendications précédentes, comprenant en outre une unité de contrôle (90) configurée pour contrôler la température du premier appareil de chauffage (65A) de sorte que la température du premier appareil de chauffage (65A) est réglée sur une première température pendant l'élution de l'acide nucléique et la température du premier appareil de chauffage (65A) devient une deuxième température plus élevée que la première température après l'élution de l'acide nucléique avant que la posture de la cartouche (1) soit changée par la rotation du corps rotatif (61) et que des gouttelettes contenant la solution d'élution introduites depuis le tube (20) dans le conteneur de réaction d'amplification d'acide nucléique (30) se déplacent vers la deuxième région.

5. Dispositif de réaction d'amplification d'acide nucléique selon l'une quelconque des revendications précédentes, comprenant en outre un mécanisme de poussée (80) qui est attaché à une ouverture du tube (20) sur un côté différent du côté communicant avec le conteneur de réaction d'amplification d'acide nucléique (30), et configuré pour expulser le liquide depuis le côté du tube (20) communicant avec le conteneur de réaction d'amplification d'acide nucléique (30) vers le conteneur de réaction d'amplification d'acide nucléique (30).

6. Procédé d'amplification d'acide nucléique mettant en oeuvre le dispositif de réaction d'amplification d'acide nucléique selon l'une quelconque des revendications précédentes, le procédé comprenant :
conduire l'acide nucléique à être élué de particules magnétiques (7) liées à l'acide nucléique dans un quatrième bouchon (47) formé d'une solution d'élution ;
rétracter les particules magnétiques (7) du quatrième bouchon (47) vers le premier bouchon (44) après l'élution de l'acide nucléique; et
effectuer un processus de formation de gouttelette pour expulser la solution d'élution du tube (20) vers le conteneur de réaction d'amplification d'acide nucléique (30) après la rétraction des particules magnétiques (7),
dans lequel la température du premier appareil de chauffage (65A) est réglée à une première température pendant l'élution de l'acide nucléique, et
la température du premier appareil de chauffage (65A) est changée pour une deuxième température de sorte que la température du premier appareil de chauffage (65A) devient la deuxième température après l'élution de l'acide nucléique avant que la posture de la cartouche (1) soit changée par la rotation du corps rotatif (61) et que des gouttelettes contenant la solution d'élution à l'intérieur du conteneur de réaction d'amplification d'acide nucléique (30) se déplacent d'une première région vers une deuxième région.

7. Procédé selon la revendication 6, dans lequel, pendant que la rétraction des particules magnétiques (7) ou le processus de formation de gouttelettes est exécuté, la température du premier appareil de chauffage (65A) est augmentée de la première température à la deuxième température.

8. Procédé selon la revendication 6 ou 7,
dans lequel l'acide nucléique est un ARN, et la solution d'élution contient un réactif de réaction de transcription inverse, et
le procédé comprend en outre :
exécuter une transcription inverse pour conduire l'ARN à être soumis à une transcription inverse dans la solution d'élution.

9. Procédé selon l'une quelconque des revendications 6 à 8, dans lequel, pendant que la rétraction des particules magnétiques (7), le processus de formation de gouttelette, ou la transcription inverse est exécutée, la température du premier appareil de chauffage (65A) est augmentée de la première température à la deuxième température.
